(19) Europäisches Patentamt
European Patent Office
Office européen des brevets

(11) EP 2 629 093 A1

(12) EUROPÄISCHE PATENTANMELDUNG

(43) Veröffentlichungstag:
21.08.2013 Patentblatt 2013/34

(51) Int Cl.:
$G01N\ 33/14^{(2006.01)}$     $G01N\ 21/55^{(2006.01)}$
$G01N\ 21/03^{(2006.01)}$     $G01N\ 21/35^{(2006.01)}$
$G01N\ 21/31^{(2006.01)}$

(21) Anmeldenummer: 13455002.9

(22) Anmeldetag: 15.02.2013

(84) Benannte Vertragsstaaten:
AL AT BE BG CH CY CZ DE DK EE ES FI FR GB
GR HR HU IE IS IT LI LT LU LV MC MK MT NL NO
PL PT RO RS SE SI SK SM TR
Benannte Erstreckungsstaaten:
BA ME

(30) Priorität: 20.02.2012 AT 2112012

(71) Anmelder: Anton Paar GmbH
8054 Graz-Straßgang (AT)

(72) Erfinder:
• Loder, Johann
8160 Weiz (AT)
• Benes, Roman
8055 Graz (AT)
• Imre, Michael
8042 Graz (AT)

(74) Vertreter: Wildhack & Jellinek
Patentanwälte
Landstraßer Hauptstraße 50
1030 Wien (AT)

(54) Verfahren und Vorrichtung zur Bestimmung des CO2-Gehalts in einer Flüssigkeit

(57) Die Erfindung betrifft ein Verfahren sowie eine Vorrichtung zur Bestimmung des $CO_2$-Gehalts ($C_{CO2}$) in einer zu prüfenden Flüssigkeit, insbesondere in einem Getränk,
- wobei die Messung der Absorption der zu messenden Flüssigkeit bei zumindest einer Wellenlänge ($\lambda_{CO2}$) innerhalb eines ersten Wellenlängenbereichs zwischen 4200 und 4300 nm durchgeführt wird und ein erster Absorptionswert ($A_{CO2}$) mittels der Methode der abgeschwächten Totalreflexion (ATR) gemessen wird,
- wobei die Messung der Absorption der zu messenden Flüssigkeit bei zumindest einer zweiten Wellenlänge ($\lambda_{ref}$) innerhalb eines zweiten Wellenlängenbereichs zwischen 3950 und 4050 nm durchgeführt wird und ein zweiter Absorptionswert ($A_{ref}$) mittels der Methode der abgeschwächten Totalreflexion (ATR) gemessen wird.
Erfindungsgemäß ist vorgesehen,
- dass die Messung der Absorption der zu messenden Flüssigkeit zusätzlich bei zumindest einer dritten Wellenlänge ($\lambda_n$) innerhalb eines dritten Wellenlängenbereichs zwischen 3300 und 3900 nm durchgeführt wird, und ein dritter Absorptionswert ($A_n$) mittels der Methode der abgeschwächten Totalreflexion (ATR) gemessen wird,
- dass eine vorgegebene Modellfunktion (M) zur Ermittlung des $CO_2$-Gehalts ($C_{CO2}$) auf Grundlage des ersten, zweiten und dritten Absorptionswerts herangezogen wird, und
- dass die Modellfunktion (M) auf den ermittelten ersten, zweiten und dritten Absorptionswerts ($A_{CO2}$, $A_{ref}$, $A_n$) angewendet wird und das Ergebnis der Auswertung als $CO_2$-Gehalt ($C_{CO2}$) der zu prüfenden Flüssigkeit zur Verfügung gehalten wird (Fig. 6).

Fig. 1

**Beschreibung**

Die Erfindung betrifft ein Verfahren zur Bestimmung des $CO_2$-Gehalts in einer zu prüfenden Flüssigkeit gemäß dem Oberbegriff des unabhängigen Patentanspruchs 1. Weiters betrifft die Erfindung eine Vorrichtung zur Bestimmung des $CO_2$-Gehalts in einer zu prüfenden Flüssigkeit gemäß dem Oberbegriff des unabhängigen Patentanspruchs 7. Vorteilhafterweise werden erfindungsgemäße Verfahren und Vorrichtungen zur Bestimmung des $CO_2$-Gehalts bei der Qualitätskontrolle von Getränken eingesetzt.

Der Anwendungsbereich der Erfindung ist jedoch nicht auf die Qualitätskontrolle von Getränken beschränkt. Die genaue Kenntnis der gelösten Inhaltsstoffe bzw. Komponenten einer zu prüfenden Flüssigkeit und deren jeweiliger Gehalt in der jeweiligen Flüssigkeit ist in vielen Produktionsbereichen wie der Biotechnologie, bei der Untersuchung von Blut und Urin usw. erforderlich. Interessierende Komponenten sind dabei beispielsweise Kohlendioxid, Methanol, Ethanol, Methan und andere chemische Substanzen, die in der Flüssigkeit, beispielsweise in einer wässrigen Lösung, enthalten sind. Eine wesentliche Anforderung der Qualitätskontrolle liegt vor allem darin, dass die Prozesse in Echtzeit kontrollierbar sein sollten. Die Messungen hierfür sollen also produktionsnah, am besten inline, erfolgen und auch in rauer Umgebung durchführbar sein.

Aus dem Stand der Technik ist eine Vielzahl von Verfahren zur Bestimmung der Konzentration von Inhaltsstoffen in Flüssigkeiten bekannt, die im Folgenden auszugsweise dargestellt werden.

Chemisch reaktive Substanzen werden häufig über Sekundäreffekte wie beispielsweise eine Umsetzung mit Säuren oder Lumineszenz-Quenching nachgewiesen. Derartige Verfahren stehen bei chemisch inaktiven Substanzen wie $CO_2$ nicht zur Verfügung. Eine Möglichkeit zur Bestimmung des Gehalts chemisch inaktiver Gase ist die Separation des zu messenden gelösten Gases durch Ausgasen in einen durch eine permeable Membran abgetrennten Messraum und anschließende Infrarotmessung des Gases, wie dies beispielsweise in der Patentschrift EP 1 630 543 offenbart ist. Solche Varianten eignen sich jedoch nur bedingt für eine Echtzeit-Anwendung, die Verwendung in einem Inline-Messverfahren ist somit nur mit großem Aufwand zu realisieren.

Daneben sind für die Ermittlung des $CO_2$-Gehalts verschiedene physikalische Nachweisverfahren gebräuchlich, beispielsweise manometrische Verfahren. Vor allem in der Brauindustrie findet auch die Volumenexpansionsmethode Anwendung, welche die gleichzeitige Messung mehrerer verschiedener gelöster Gase aufgrund der gemessenen Druck- und Temperaturwerte ermöglicht. Dieses Verfahren ist eingehend in der Patentschrift AT 409 673 erläutert.

Ein weiteres Verfahren beruht auf der Auswertung von Absorptions- bzw. Transmissionsspektren, in denen die Anregung von charakteristischen Molekülschwingungen, dies sind Rotation und/oder Vibration, in der Flüssigkeit zu einer Energieabsorption und damit einer Intensitätsänderung im anregenden Spektrum führt. Mit diesem Verfahren können Inhaltsstoffen geringer und geringster Konzentration ermittelt werden, wobei aus der Absorption von Infrarotstrahlung die jeweilige Konzentration des Inhaltsstoffs in festen, flüssigen oder gasförmigen Medien ermittelt wird. Je nach Messaufgabe werden unterschiedliche Wellenlängenbereiche des Spektrums für die Strukturaufklärung eingesetzt, der Messbereich reicht von UV/VIS- bis hin in den Infrarotbereich. Aus der Absorption von Strahlung bestimmter Energie kann auf die angeregten Molekül- bzw. Gitterschwingungen und damit auf die Bestandteile des untersuchten Materials rückgeschlossen werden. Für die Messstrahlung hinreichend durchlässige Substanzen können in Transmission vermessen werden, für opake Festkörper und für stark gefärbte Lösungen ist die Untersuchung der Reflexion wie beispielsweise mit der Methode der abgeschwächten Totalreflexion (ATR) bekannt. In der Prozessanalytik ist die Anwendbarkeit von Transmissionsmessungen häufig durch die starke Absorption durch Wassermoleküle im infraroten Bereich begrenzt, sodass Reflexionsmessungen wie die Methode der ATR vorteilhaft zur Anwendung gelangen. Die spektroskopische Bestimmung zeigt auch den Vorteil, dass die Messergebnisse unabhängig vom Druck der untersuchten Flüssigkeit sowie deren Komponenten sind.

Die untersuchten Infrarotspektren können also zur Strukturaufklärung verwendet werden, bei bekannter Zusammensetzung der untersuchten Flüssigkeit kann auch die Konzentration der untersuchten Komponenten in der Flüssigkeit festgestellt werden. Besitzen die untersuchten Flüssigkeiten zu hohe Absorptionswerte um ein verwertbares Signal zu erhalten, wird häufig auf das Prinzip der abgeschwächten Totalreflexion (ATR, attenuated total reflection) zurückgegriffen. Im Infrarotbereich gibt es mehrere in Frage kommende Absorptionsbanden die den $CO_2$-Schwingungen zugeordnet werden können. Gelöstes $CO_2$ besitzt beispielsweise im Bereich um 4,3 $\mu$m ein charakteristisches Absorptionsband, das von den in üblichen untersuchten Getränkeproben vorkommenden Komponenten weitgehend unabhängig ist.

Die Technik der ATR, die auch unter der Bezeichnung mehrfache innere Reflexion bekannt ist, wird seit vielen Jahren zu Analysezwecken verwendet. In der ATR-Spektroskopie wird der Effekt ausgenutzt, dass ein Lichtstrahl an der Grenzfläche zwischen einem optisch dichterem Medium mit dem Brechungsindex $n_1$ und einem optisch dünneren Medium mit dem Brechungsindex $n_2$ ($n_1 > n_2$) total reflektiert wird, wenn der Einfallswinkel des Lichtstrahls zur Grenzfläche hin den Grenzwinkel $\theta$ der Totalreflexion überschreitet. Für den Grenzwinkel $\theta$ gilt: $\sin(\theta) = n_2/n_1$. An der Grenzfläche tritt der Lichtstrahl ins optisch dünnere Medium aus und wechselwirkt mit diesem. Hinter der reflektierenden Fläche bildet sich die sogenannte evaneszente Welle mit einer Eindringtiefe $d_p$ im Bereich der Wellenlänge $\lambda$. Die Eindringtiefe

$d_p$ hängt dabei von den beiden Brechungsindizes $n_1$ und $n_2$, der verwendeten Wellenlänge $\lambda$ und dem Einfallswinkel $\theta$ ab.

$$d_p = \frac{\lambda}{2 \cdot \pi \cdot \sqrt{n_1^2 \cdot sin^2(\theta) - n_2^2}}$$

Absorbiert das optisch dünnere Medium die eindringende Strahlung, wird der totalreflektierte Strahl geschwächt. Die Absorption ist also abhängig von der Wellenlänge $\lambda$ und das Spektrum der totalreflektierten Strahlung kann analog zur Transmissionsmessung für die spektroskopische Auswertung herangezogen werden. Aus dem Transmissions- bzw. Extinktionsspektrum kann auf die Zusammensetzung des optisch dünneren Medium geschlossen werden.

Es ist allgemein bekannt Absorptionsspektren zu Zwecken des Nachweises von Inhaltsstoffen in Flüssigkeiten sowie zur Charakterisierung von Flüssigkeitsgemischen einzusetzen, wobei Inhaltsstoffen bereits bei geringsten Konzentrationen detektierbar und quantifizierbar sind. Hierbei wird ausgenutzt, dass Moleküle durch Infrarotstrahlungen ausgewählter Wellenlänge in Schwingung versetzt werden, gelöstes $CO_2$ beispielsweise besitzt im Bereich um 4,3 $\mu$m ein charakteristisches Absorptionsband. Über das Lambert-Beer'sche Gesetz kann die Absorption in präzise Konzentrationsmessungen umgesetzt werden. Es beschreibt

$$E_\lambda = -\lg\left(\frac{I}{I_0}\right) = \varepsilon_\lambda \cdot c \cdot d$$

wobei $E_\lambda$, der Extinktion, I der Intensität des transmittierten Lichtes, $I_0$ der Intensität des einfallenden Lichtes, $\varepsilon_\lambda$ dem Extinktionskoeffizienten, c Konzentration des gelösten $CO_2$ und d der Schichtdicke des durchstrahlten Körpers / fluiden Mediums entspricht.

Kernstück des ATR-Sensors ist ein ATR-Reflexionselement, das im interessierenden Bereich für die Messstrahlung transparent ist und einen großen Brechungsindex aufweist. Bekannte Materialien für ATR-Reflexionselemente sind beispielsweise Saphir, ZnSe, Ge, SI, Thalliumbromid, YAG(Yttrium Aluminium Granat $Y_3Al_5O_{12}$, Spinell ($MgAl_2O_4$) usw. Häufig werden diese Reflexionselemente so ausgeführt, dass an der Grenzfläche zur Flüssigkeit die Interaktionslänge durch Mehrfachreflexionen erhöht wird. Weitere Elemente sind ein oder mehrere Strahlungsquellen passender Frequenz (bereiche), gegebenenfalls mit Mitteln zur Frequenzauswahl, und ein oder mehrere Detektoren, auch diese können frequenzselektiv ausgeführt sein.

In der einfachsten Form umfasst ein ATR-Sensor ein ATR-Reflexionselement, das die internen Reflexionen ermöglicht, eine Strahlungsquelle und eine Detektionseinheit. Das ATR-REflexionselementragt dabei in eine zu untersuchende Flüssigkeit, entweder direkt in den Prozessstrom oder in die zu untersuchende Substanz in einem Behälter hinein. Häufig wird dabei eine zweite Frequenz untersucht, um auf die Absorption des Lösungsmittels zu referenzieren. Dies passiert entweder durch passende Mittel zur Frequenzauswahl (z.B. variable Filter) oder geteilte Filterbereiche am Detektor oder durch eine zweite Anordnung von Quelle und Detektor am selben ATR-Reflelexionselement. Das Reflexionselement ist dabei dicht gegen das Gehäuse gepresst und hermetisch dicht verbunden, wobei unterschiedliche Dichtmaterialien je nach geforderter chemischer Beständigkeit und Druckfestigkeit Verwendung finden.

Aus dem Stand der Technik ist bekannt, dass solche optischen Messsysteme für die Prozessüberwachung jeweils an die zu untersuchenden Flüssigkeiten angepasst werden. Die tatsächlich am ATR-Sensor bestimmten Absorptionswerte werden durch Wahl eines an die jeweilige Flüssigkeit angepassten Kalibriermodells in Konzentrationen umgerechnet, wobei eine Vielzahl von bekannten, die Messung beeinflussenden Größen bei der Kalibrierung berücksichtigt werden. Ausgehend von an bekannten Zusammensetzungen gemessenen Kalibrierkurven werden die gemessenen Absorptionswerte in tatsächliche Konzentrationswerte nach den oben beschriebenen Gesetzen umgewandelt.

Gemäß Lambert-Beer'schen Gesetz hängt die tatsächliche absorbierte Intensität vom wellenlängenabhängigen Extinktionskoeffizient sowie von der Konzentration c der absorbierenden Komponente und der Schichtdicke d des durchstrahlten Körpers ab. In ATR-Geometrie ist die Schichtdicke der untersuchten Lösung aber durch die Eindringtiefe $d_p$ der evaneszenten Welle bestimmt. Wie tief diese in das optisch dünnere Medium eindringt, hängt gemäß den Gesetzen für die Totalreflexion vom Brechungsindex des Reflexionselements sowie von der jeweiligen Flüssigkeit ab.

Der Brechungsindex der Lösung spielt somit insofern eine Rolle bei der $CO_2$-Bestimmung von Getränken, als die Eindringtiefe des ATR-Strahls und somit auch die Absorption des Strahls ganz wesentlich vom Verhältnis der Brechungsindizes des ATR-Reflexionselements und der zu prüfenden Flüssigkeit abhängt. Dieser Brechungsindex der zu prüfenden Flüssigkeit wird zumeist überwiegend durch den Zucker-, Extrakt und/oder Alkoholgehalt der Flüssigkeit bestimmt, sodass die Art der Flüssigkeit selbst wesentlichen Einfluss auf die Absorption des ATR-Strahls hat.

Aufgrund des unterschiedlichen Brechungsindex von zu untersuchenden Flüssigkeiten und der daraus resultierenden Absorption besteht somit das Problem, dass für jeweils unterschiedliche Flüssigkeiten jeweils unterschiedliche Kalibriermodelle gefunden werden müssen, um eine Bestimmung des $CO_2$-Gehalts zu ermöglichen. Für jede Flüssigkeit muss separat jeweils ein Kalibriermodell gefunden werden.

Wird beispielsweise eine Abfüllanlage für eine Vielzahl von unterschiedlichen Flüssigkeiten verwendet, so besteht das Problem, dass bei Wechsel der abzufüllenden Flüssigkeit jeweils eine Kalibrierung vorgenommen oder zumindest das jeweilige Kalibriermodell von Hand umgestellt werden muss. Dies kann insbesondere im raschen Wechsel zwischen einzelnen Flüssigkeiten oder bei sehr großen Anlagen mit vielen zugleich abgefüllten Flüssigkeiten zu Problemen führen, insbesondere dann, wenn die jeweilige Flüssigkeit falsch ausgewählt wird.

Die Aufgabe der Erfindung besteht somit darin, die vorstehend genannten Probleme zu überwinden und die $CO_2$-Konzentration unabhängig von einem voreingestellten, speziell auf die jeweilige Flüssigkeit abgestimmten Kalibriermodell zu ermitteln und so eine fehleranfällige Vorwahl der jeweiligen Flüssigkeit zu vermeiden. Die Messung und Auswertung der Messwerte soll damit unabhängig von einer allfälligen Vorwahl der Flüssigkeit durchgeführt werden, Fehlereinflüsse durch falsche Modellwahl und/oder sich ändernde Produktzusammensetzungen sollen vermieden werden.

Die Erfindung löst diese Aufgabe bei einem Verfahren der eingangs genannten Art mit den kennzeichnenden Merkmalen des Patentanspruchs 1.

Erfindungsgemäß ist bei einem Verfahren zur Bestimmung des $CO_2$-Gehalts in einer zu prüfenden Flüssigkeit, insbesondere in einem Getränk, wobei die Messung der Absorption der zu messenden Flüssigkeit bei zumindest einer Wellenlänge innerhalb eines ersten Wellenlängenbereichs zwischen 4200 und 4300 nm durchgeführt wird und ein erster Absorptionswert mittels der Methode der abgeschwächten Totalreflexion gemessen wird, wobei die Messung der Absorption der zu messenden Flüssigkeit bei zumindest einer zweiten Wellenlänge innerhalb eines zweiten Wellenlängenbereichs zwischen 3950 und 4050 nm durchgeführt wird und ein zweiter Absorptionswert mittels der Methode der abgeschwächten Totalreflexion gemessen wird, vorgesehen, dass die Messung der Absorption der zu messenden Flüssigkeit zusätzlich bei zumindest einer dritten Wellenlänge innerhalb eines dritten Wellenlängenbereichs zwischen 3300 und 3900 nm durchgeführt wird, und ein dritter Absorptionswert mittels der Methode der abgeschwächten Totalreflexion gemessen wird, dass eine vorgegebene Modellfunktion zur Ermittlung des $CO_2$-Gehalts auf Grundlage des ersten, zweiten und dritten Absorptionswerts herangezogen wird, und dass die Modellfunktion auf den ermittelten ersten, zweiten und dritten Absorptionswerts angewendet wird und das Ergebnis der Auswertung als $CO_2$-Gehalt der zu prüfenden Flüssigkeit zur Verfügung gehalten wird. Durch diese Vorgehensweise ist es nicht mehr erforderlich für jede zu prüfende Flüssigkeit eine separate Kalibrierung vorzunehmen und es ist auch nicht mehr erforderlich, zwischen dem Wechsel der Art der zu prüfenden Flüssigkeit jeweils Änderungen des Kalibriermodells vorzunehmen oder das Kalibriermodell zu ändern.

Zur numerisch stabilen Bestimmung des $CO_2$-Gehalts sowie zur einfachen Ermittlung der jeweiligen Absorption kann vorgesehen sein, dass die Messung des ersten Absorptionswerts durch Bestimmung der absorbierten Intensität in einem ersten Messbereich vorgenommen wird, der durch eine im ersten Wellenlängenbereich liegende erste Schwerpunktswellenlänge sowie eine erste Bereichsbreite $2\Delta\lambda_{CO2}$ festgelegt ist, wobei der erste Messbereich im Bereich von $\lambda_{S,CO2} \pm \Delta\lambda_{CO2}$ liegt, und/oder dass die Messung des zweiten Absorptionswerts durch Bestimmung der absorbierten Intensität in einem zweiten Messbereich vorgenommen wird, der durch eine im zweiten Wellenlängenbereich liegende zweite Schwerpunktswellenlänge sowie eine zweite Bereichsbreite $2\Delta\lambda_{ref}$ festgelegt ist, wobei der zweite Messbereich im Bereich von $\lambda_{S,ref} \pm \Delta\lambda_{ref}$ liegt, und/oder dass die Messung des dritten Absorptionswerts durch Bestimmung der absorbierten Intensität in einem dritten Messbereich vorgenommen wird, der durch eine im dritten Wellenlängenbereich liegende dritte Schwerpunktswellenlänge sowie eine dritte Bereichsbreite $2\Delta\lambda_n$ festgelegt ist, wobei der dritte Messbereich im Bereich von $\lambda_{S,n} \pm \Delta\lambda_n$ liegt, wobei die erste und/oder zweite und/oder dritte Bereichsbreite jeweils in einem Bereich zwischen 20 nm und 200 nm, insbesondere bei 100 nm liegt.

Alternativ kann zum selben Zweck vorgesehen sein, dass der erste Absorptionswert, vorzugsweise ausschließlich, bei einer vorgegebenen im ersten Wellenlängenbereich liegenden ersten Wellenlänge, von vorzugsweise 4260 nm, ermittelt wird, und/oder dass der zweite Absorptionswert, vorzugsweise ausschließlich, bei einer vorgegebenen im zweiten Wellenlängenbereich liegenden zweiten Wellenlänge, von vorzugsweise 4020 nm, ermittelt wird, und/oder dass der dritte Absorptionswert, vorzugsweise ausschließlich, bei einer vorgegebenen im dritten Wellenlängenbereich liegenden dritten Wellenlänge, von vorzugsweise 3800 nm, ermittelt wird.

Um die Einflüsse der Temperatur auf die Absorption besser berücksichtigen zu können, kann vorgesehen sein, dass zusätzlich zur Bestimmung des ersten, zweiten und dritten Absorptionswerts auch die Temperatur der zu prüfenden Flüssigkeit ermittelt wird, dass die Modellfunktion zur Ermittlung des $CO_2$-Gehalts neben dem ersten, zweiten und dritten Absorptionswert auch die Temperatur der zu prüfenden Flüssigkeit berücksichtigt, und dass die Modellfunktion auf den ermittelten ersten, zweiten und dritten Absorptionswerts sowie auf die ermittelte Temperatur angewendet wird und das Ergebnis der Auswertung als $CO_2$-Gehalt der zu prüfenden Flüssigkeit zur Verfügung gehalten wird.

Zur Kalibrierung und zur Ermittlung einer vorteilhaften Modellfunktion kann vorgesehen sein, dass vor der

Bestimmung des $CO_2$-Gehalts eine Modellfunktion erstellt und für die Bestimmung des $CO_2$-Gehalts zur Verfügung gehalten wird, indem eine Vielzahl von Referenzmessungen des ersten, zweiten und dritten Absorptionswerts für jeweils unterschiedliche Referenzflüssigkeiten mit bekanntem $CO_2$-Gehalt mit unterschiedlichem, gegebenenfalls bekannten Brechungsindex, durchgeführt werden, mittels eines Fitting-Verfahrens die Modellfunktion der Form

$$M = M(\mathbf{A}_{CO2}, \mathbf{A}_{ref}, \mathbf{A}_n, ..., B_1, ..., B_N)$$

erstellt wird, wobei vorab unbekannte Modellparameter an den jeweils vorgegebenen $CO_2$-Gehalt sowie die ermittelten ersten, zweiten und dritten Absorptionswerte angepasst werden, sodass man bei Anwendung der Modellfunktion auf die ersten, zweiten und dritten Absorptionswerte jeweils, zumindest näherungsweise, den bekannten $CO_2$-Gehalt erhält.

Um hierbei die Einflüsse der Temperatur mit berücksichtigen zu können, kann vorgesehen sein, dass bei der Vielzahl von Referenzmessungen jeweils neben dem ersten, zweiten und dritten Absorptionswert auch die Temperatur der jeweiligen Referenzflüssigkeit ermittelt wird, mittels eines Fitting-Verfahrens die Modellfunktion der Form

$$M = M(\mathbf{A}_{CO2}, \mathbf{A}_{ref}, \mathbf{A}_n, T, C_1, ..., C_N)$$

erstellt wird, wobei vorab unbekannte Modellparameter an den jeweils vorgegebenen $CO_2$-Gehalt, die ermittelten ersten, zweiten und dritten Absorptionswerte sowie die jeweilige Temperatur angepasst werden, sodass man bei Anwendung der Modellfunktion auf die ersten, zweiten und dritten Absorptionswerte sowie die Temperatur jeweils, zumindest näherungsweise, den bekannten $CO_2$-Gehalt erhält.

Die Erfindung löst diese Aufgabe bei einem Verfahren der eingangs genannten Art mit den kennzeichnenden Merkmalen des Patentanspruchs 7.

Erfindungsgemäß ist bei einer Vorrichtung zur Bestimmung des $CO_2$-Gehalts in einer zu prüfenden Flüssigkeit, umfassend eine erste ATR-Messeinheit zur Bestimmung eines ersten Absorptionswerts bei zumindest einer ersten Wellenlänge innerhalb eines ersten Wellenlängenbereichs zwischen 4200 und 4300 nm, eine zweite ATR-Messeinheit zur Bestimmung eines zweiten Absorptionswerts bei zumindest einer zweiten Wellenlänge innerhalb eines zweiten Wellenlängenbereichs zwischen 3950 und 4050 nm, vorgesehen:

- eine dritte ATR-Messeinheit zur Bestimmung eines dritten Absorptionswerts bei einer zumindest dritten Wellenlänge innerhalb eines dritten Wellenlängenbereichs zwischen 3300 und 3900 nm, und
- eine Auswerteeinheit, die den ATR-Messeinheiten nachgeschaltet ist und der die Resultate der ATR-Messeinheiten zugeführt sind, wobei die Auswerteeinheit eine Modellfunktion auf den ersten, zweiten und dritten Absorptionswert anwendet und das Ergebnis der Auswertung an ihrem Ausgang als $CO_2$-Gehalt der zu prüfenden Flüssigkeit zur Verfügung hält.

Zur numerisch stabilen Bestimmung des $CO_2$-Gehalts kann vorgesehen sein, dass die ATR-Messeinheiten für die Bestimmung der absorbierten Intensität in einem ersten, zweiten und dritten Messbereich sensitiv sind, wobei der erste Messbereich durch eine im ersten Wellenlängenbereich liegende erste Schwerpunktswellenlänge sowie eine erste Bereichsbreite $2\Delta\lambda_{CO2}$ festgelegt ist, und der erste Messbereich im Bereich von $\lambda_{S,CO2} \pm \Delta\lambda_{CO2}$ festgelegt ist, und/oder wobei der zweite Messbereich durch eine im zweiten Wellenlängenbereich liegende zweite Schwerpunktswellenlänge sowie eine zweite Bereichsbreite $2\Delta\lambda_{ref}$ festgelegt ist, und der zweite Messbereich im Bereich von $\lambda_{S,ref} \pm \Delta\lambda_{ref}$ festgelegt ist, und/oder wobei der dritte Messbereich durch eine im dritten Wellenlängenbereich liegende dritte Schwerpunktswellenlänge sowie eine dritte Bereichsbreite $2\Delta\lambda_n$ festgelegt ist, und der dritte Messbereich im Bereich von $\lambda_{S,n} \pm \Delta\lambda_n$ festgelegt ist, wobei die erste und/oder zweite und/oder dritte Bereichsbreite jeweils in einem Bereich zwischen 20 nm und 200 nm, insbesondere bei 100 nm liegt.

Um die Einflüsse der Temperatur auf die Absorption und somit auf den ermittelten $CO_2$-Gehalt besser berücksichtigen zu können, kann eine der Auswerteeinheit vorgeschalteten Temperaturfühler zur Bestimmung der Temperatur der zu prüfenden Flüssigkeit vorgesehen sein, wobei die Auswerteeinheit eine Modellfunktion auf den ersten, zweiten und dritten Absorptionswert sowie auf die vom Temperaturfühler ermittelte Temperatur anwendet und das Ergebnis der Auswertung an ihrem Ausgang als $CO_2$-Gehalt der zu prüfenden Flüssigkeit zur Verfügung hält.

Zur vorteilhaften Aufbewahrung oder Weiterleitung der zu prüfenden Flüssigkeit während der Qualitätsprüfung kann ein Behälter zur Aufbewahrung oder Durchleitung der zu prüfenden Flüssigkeit, vorgesehen sein, wobei die sensitiven Oberflächenteile der ATR-Messeinheiten und gegebenenfalls auch des Temperaturfühlers bei Befüllen oder Durchströmen des Behälters mit der zu prüfenden Flüssigkeit mit dieser in Kontakt treten und insbesondere im Inneren des Gefäßes angeordnet sind.

Um das Kalibriermodell rasch abrufen und auf die ermittelten Messwerte anwenden zu können, kann vorgesehen sein, dass in der Auswerteeinheit Speicher für vorgegebene Koeffizienten vorgesehen sind, und dass die Auswerteeinheit eine Recheneinheit aufweist, der die abgespeicherten Koeffizienten sowie der erste, zweite und dritte Absorptionswert, sowie gegebenenfalls auch die ermittelte Temperatur, zugeführt sind und die auf Grundlage der ihr zugeführten Werte die Modellfunktion auswertet und am Ausgang der Auswerteeinheit zur Verfügung hält.

Vorteilhafte platzsparende Weiterbildungen der Erfindung sehen vor, dass jede ATR-Messeinheit jeweils ein ATR-Reflexionselement, eine ATR-Infrarotquelle, und einen ATR-Infrarotsensor umfassen, oder dass die ATR-Messeinheiten ein gemeinsames ATR-Reflexionselement und eine gemeinsame im ersten, zweiten und dritten Wellenlängenbereich aktive ATR-Infrarotquelle aufweisen und einen gemeinsamen, im ersten, zweiten und dritten Wellenlängenbereich aktiven ATR-Infrarotsensor aufweisen, wobei im Strahlengang zwischen der ATR-Infrarotquelle und dem ATR-Infrarotsensor ein einstellbarer Filter, insbesondere ein Filterrad oder ein Fabry-Perot-Interferometer, vorgesehen ist, der je nach seiner Einstellung jeweils nur für Strahlung im ersten, zweiten oder dritten Wellenlängenbereich durchlässig ist, oder dass die ATR-Messeinheiten ein gemeinsames ATR-Reflexionselement und eine gemeinsame im ersten, zweiten und dritten Wellenlängenbereich aktive ATR-Infrarotquelle aufweisen und für den ersten, zweiten und dritten Wellenlängenbereich jeweils separate, am Ende des jeweiligen Strahlengangs befindliche ATR-Infrarotsensoren vorgesehen sind, oder dass die ATR-Messeinheiten ein gemeinsames ATR-Reflexionselement und einen gemeinsamen für alle Wellenlängenbereiche sensitiven ATR-Infrarotsensor aufweisen, und für den ersten, zweiten und dritten Wellenlängenbereich jeweils separate, ATR-Infrarotquellen vorgesehen sind.

Eine platzoptimierte Ausführungsform der Erfindung mit einem einzigen Reflexionselement sieht vor, dass die ATR-Messeinheiten zumindest zwei separate ATR-Infrarotquellen und zugehörige ATR-Infrarotsensoren aufweisen, die jeweils voneinander unabhängige Strahlengänge besitzen und für jeweils zwei Messbereiche verschieden sensitiv sind, wobei jeweils eine Messeinheit des ersten Infrarotsensors und jeweils eine Messeinheit des zweiten Infrarotsensors jeweils für denselben Wellenlängenbereich sensitiv sind und eine Referenzierungseinheit vorgesehen ist, die den Messwert der dritten Messeinheit mit dem Verhältnis der Messwerte der beiden für denselben Wellenlängenbereich sensitiven Messeinheiten multipliziert und an ihrem Ausgang zur Verfügung hält.

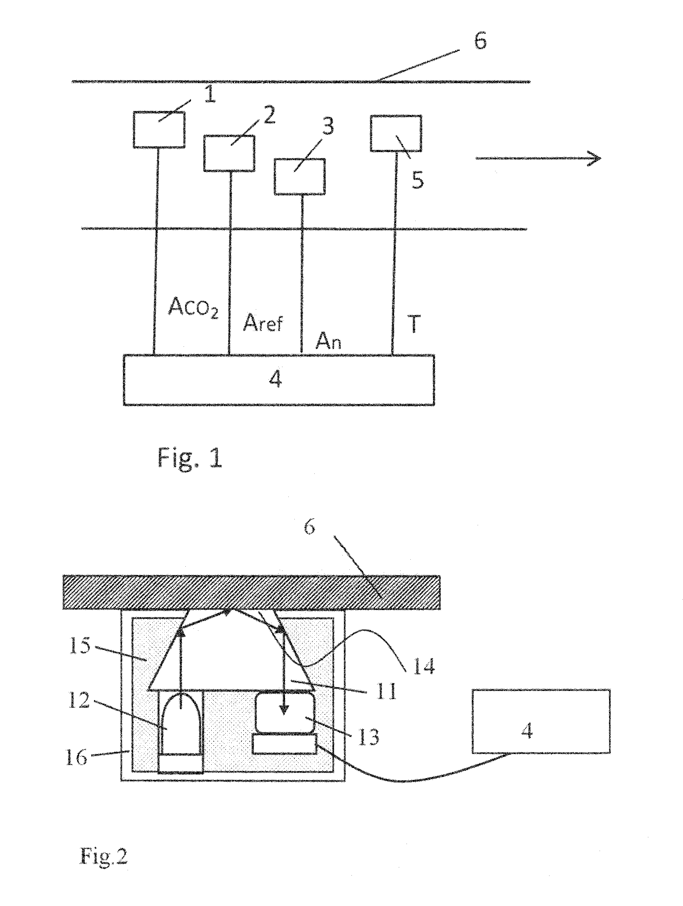

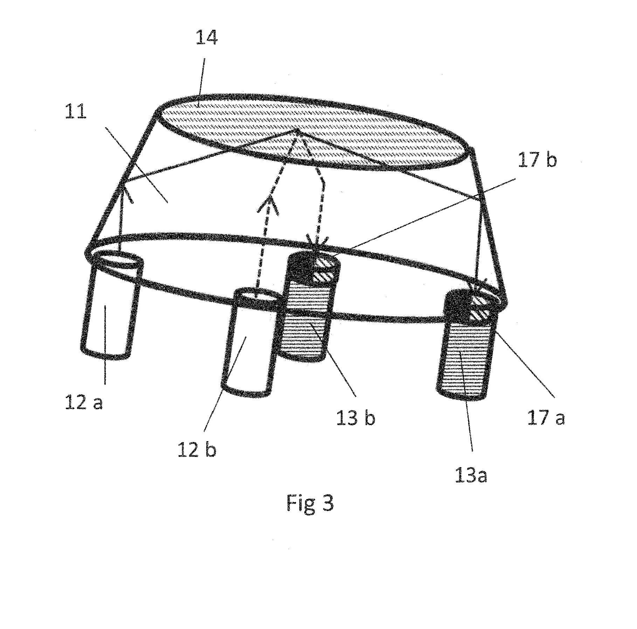

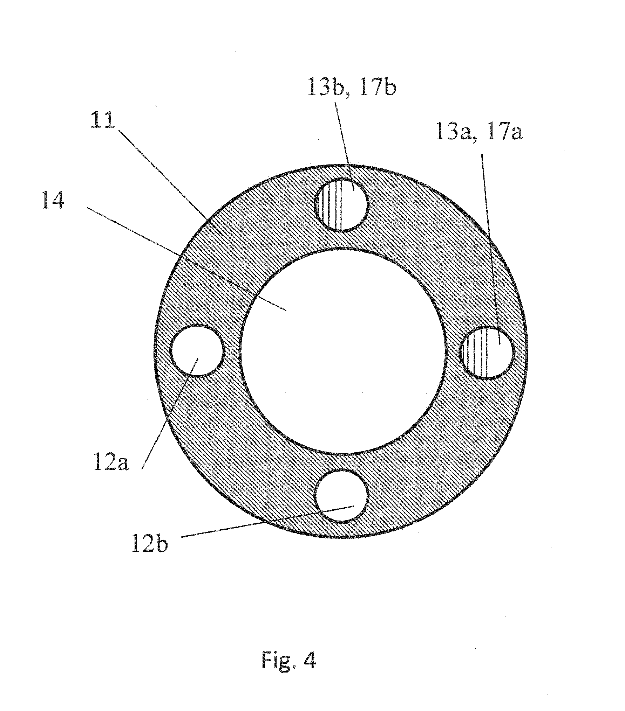

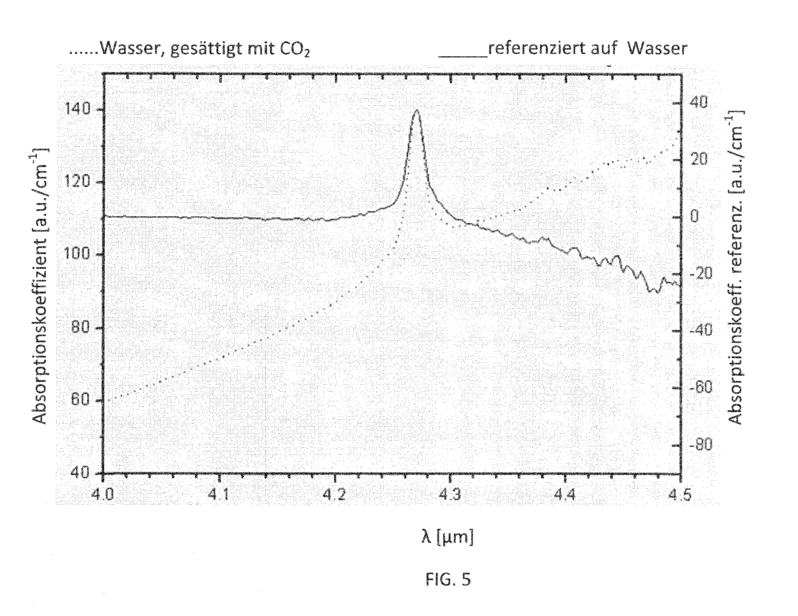

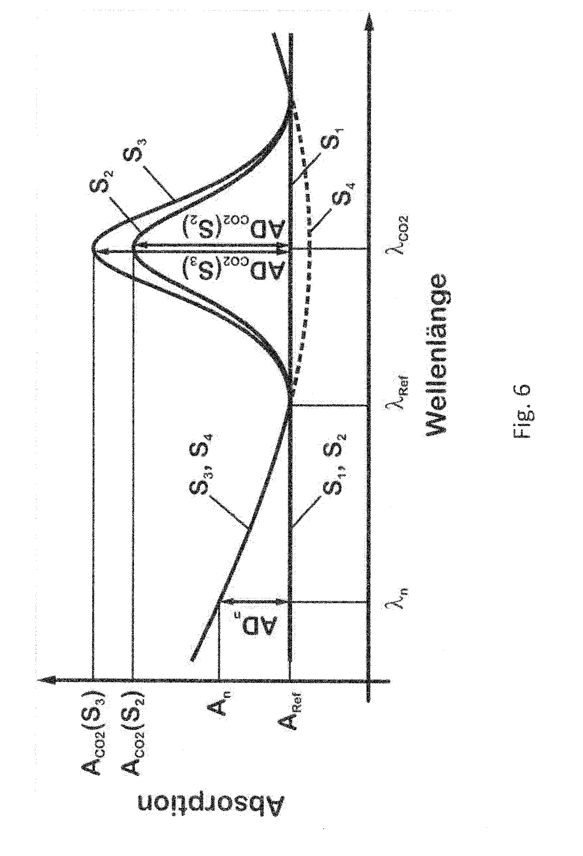

Die Erfindung wird anhand eines konkreten Ausführungsbeispiels unter Zuhilfenahme der folgenden Zeichnungsfiguren erläutert. **Fig. 1** eine Ausführungsform einer erfindungsgemäßen Vorrichtung. **Fig. 2** zeigt den Aufbau eines ATR-Sensors im Detail. **Fig. 3** zeigt einen bevorzugten ATR-Sensor in Schrägansicht. **Fig. 4** zeigt den in **Fig. 3** dargestellten ATR-Sensor in Vorderansicht. **Fig. 5** zeigt das Spektrum einer zu prüfenden Flüssigkeit. **Fig. 6** zeigt schematisch Spektren von zu prüfenden Flüssigkeiten jeweils mit unterschiedlichem $CO_2$-Gehalt und unterschiedlichem Brechungsindex referenziert auf das Lösungsmittel.

In der vorliegenden in **Fig. 1** dargestellten Ausführungsform der Erfindung wird die zu prüfende Flüssigkeit durch einen Behälter 6 in Form einer Rohrleitung durchgeleitet. An den der Innenoberfläche der Rohrleitung sind drei separate ATR-Messeinheiten 1, 2, 3 sowie ein Temperaturfühler 5 angeordnet. Die sensitiven Oberflächenteile 14 (siehe Fig.2) der ATR-Messeinheiten 1, 2, 3 stehen in Kontakt mit der durch die Rohrleitung geleiteten Flüssigkeit. Der Temperaturfühler 5 befindet sich im Inneren der Rohrleitung oder an der Begrenzungswand mit Kontakt zur Flüssigkeit. Die Messergebnisse des Temperaturfühlers 5 sowie der ATR-Messeinheiten 1, 2, 3 sind einer Auswerteeinheit 4 zugeführt.

In **Fig. 2** ist eine ATR-Messeinheit 1, 2, 3 im Detail dargestellt. Kernstück der ATR-Messeinheit 1, 2, 3 ist ein Reflexionselement 11, das im interessierenden Wellenlängenbereich für Strahlung transparent ist und einen hohen Brechungsindex aufweist.

Diese Elemente können beispielsweise ein Prisma, ein spezieller ATR-Kristall, ein Lichtwellenleiter usw. sein. Bekannte Materialien für solche optischen Elemente sind beispielsweise Saphir, ZnSe, Ge, SI, Thalliumbromid, YAG und Spinell usw. Häufig werden die Reflexionselemente 11 so ausgeführt, dass in ihrem Inneren über Mehrfachreflexionen die Intensitätsausbeute erhöht wird. Weiters umfasst die ATR-Messeinheit 1, 2, 3 eine Strahlungsquelle 12 im jeweiligen Frequenzbereich und einen Detektor 13. Am Ausgang des Detektors 13 liegt jeweils ein Messsignal an, das der vom Detektor 13 ermittelten Intensität entspricht. Beliebige frequenzselektive Mittel im Strahlengang zwischen Quelle und Detektor legen die Messwellenlänge $\lambda$ bzw. den Messbereich $\lambda_S \pm \Delta\lambda$ rund um die Schwerpunktswellenlänge $\lambda_S$ der ATR-Messeinheit fest.

Jede der ATR-Messeinheiten 1, 2, 3 ist im vorliegenden Ausführungsbeispiel von einem Gehäuse 16 umgeben, das einen Gehäuseinnenraum 15 festlegt, in dem das Reflexionselement 11, die Strahlungsquelle 12 und der Detektor 13 angeordnet sind. Der sensitive Oberflächenteil 14 des Reflexionselements 11 setzt die Außenwand des Gehäuses 16 gegenüber der zu prüfenden Flüssigkeit fort und steht mit der zu prüfenden Flüssigkeit in Berührung. Das Reflexionselement 11 ist dabei dicht gegen das Gehäuse 16 gepresst oder mit diesem druck- und gasdicht verbunden, beispielsweise über einen O-Ring oder mittels Lötverbindung oder auch durch unelastische Dichtungen, etwa aus PEEK oder TEFLON.

Alternativ können auch die einzelnen ATR-Messeinheiten 1, 2, 3 in einem gemeinsamen Gehäuse 16 integriert sein und einen gemeinsames Reflexionselement 11 und insbesondere auch eine gemeinsame Strahlungsquellen 12 oder einen gemeinsamen Detektor 13 aufweisen. Hierbei haben sich die im Folgenden dargestellten Ausführungsformen als zweckmäßig erwiesen:

Bei einer ersten Alternative weisen die ATR-Messeinheiten 1, 2, 3 einen gemeinsamen ATR-Reflexionselement 11 und eine gemeinsame im ersten, zweiten und dritten Wellenlängenbereich aktive ATR-Infrarotquelle 12 auf. Weiters weisen die ATR-Messeinheiten 1, 2, 3 auch einen gemeinsamen, im ersten, zweiten und dritten Wellenlängenbereich aktiven ATR-Infrarotsensor auf. Im Strahlengang zwischen der Infrarotquelle 11 und dem Infrarotsensor 12 ist ein einstellbarer Filter mit verstellbarer Filtercharakteristik angeordnet. Ein solcher Filter ist vorteilhafterweise in Form eines Filterrads ausgestaltet, das an unterschiedlichen Umfangsbereichen Filter mit unterschiedlicher Filtercharakteristik aufweist. Das Filterrad wird von einem Motor gedreht, sodass sich je nach Einstellung des Filterrads vorgegebener Umfangsbereich mit seiner jeweiligen Filtercharakteristik in den Strahlengang befindet. Jeder Umfangsbereich weist einen Filter auf, der jeweils nur im ersten, zweiten oder dritten Wellenlängenbereich durchlässig ist, sodass je nach Einstellung des Filters die ATR-Messeinheit 1, 2, 3 jeweils Messungen der Absorption im ersten, zweiten oder dritten Wellenlängenbereich vornimmt. Ein solcher Filter kann auch nach Art eines Fabry-Perot-Interferometers ausgebildet sein,

Bei einer zweiten Alternative weisen die ATR-Messeinheiten 1, 2, 3 ein gemeinsames Reflexionselement 11 und eine gemeinsame im ersten, zweiten und dritten Wellenlängenbereich aktive Infrarotquelle 12 auf. Für den ersten, zweiten und dritten Wellenlängenbereich sind jeweils separate, am Ende des jeweiligen Strahlengangs befindliche Infrarotsensoren 13 vorgesehen.

Bei einer dritten Alternative weisen die ATR-Messeinheiten 1, 2, 3 ein gemeinsames Reflexionselement 11 und einen gemeinsamen für alle Wellenlängenbereiche sensitiven Infrarotsensor 13 auf, wobei für den ersten, zweiten und dritten Wellenlängenbereich jeweils separate, Infrarotquellen 12 vorgesehen sind.

Eine weitere Alternative stellen Kombinationen der oben genannten Aufbauten dar. Beispielsweise kann ein ATR-Reflexionselement mit jeweils 2 Detektoren und Quellen in 2 unterschiedlichen Wellenlängenbereichen ausgestattet sein, die jeweils einen geteilten Filter zur zusätzlichen Messung einer weiteren Frequenz, beispielsweise einer Referenzfrequenz verwenden. Auf diese Art können auch bei Schwankungen der Quelle unterschiedliche Intensitäten aufeinander normiert werden.

Die jeweiligen Messsignale der ATR-Messeinheiten 1, 2, 3 sowie der am Ausgang der Temperaturmesseinheit 5 anliegende Temperaturwert **T** werden wird Auswerteeinheit 4 zugeführt, die, wie nachstehend beschrieben, einen Wert für die $CO_2$-Konzentration ermittelt und an ihrem Ausgang für die weitere Verwendung zur Verfügung hält. Dieser Wert für die $CO_2$-Konzentration kann für die Einstellung der gewünschten Konzentration durch Anpassung der $CO_2$-Zufuhr in den Prozess herangezogen werden. Ebenso kann der Wert für die $CO_2$-Konzentration dazu herangezogen werden, Teile der Flüssigkeit, mit zu hoher oder zu geringer $CO_2$-Konzentration umzuleiten und auszusondern oder in den Produktionsprozess zurückzuführen.

In **Fig. 3** ist eine bevorzugte Ausführungsform eines ATR-Sensors mit vier Messeinheiten 1, 2a, 2b, 3 dargestellt, mit dem drei Absorptionswerte $A_{CO2}$, $A_{ref}$, $A_n$ ermittelt werden. In dieser besonderen Ausführungsform ist ein einziges Reflexionselement 11 vorgesehen, wobei zwei Infrarotquellen 12 vorgesehen sind, von denen die erste Infrarotquelle 12a Infrarotlicht im ersten und zweiten Wellenlängenbereich und die zweite Infrarotquelle 12a Infrarotlicht im zweiten und dritten Wellenlängenbereich abstrahlt. Weiters sind zwei Infrarotsensoren 13 vorgesehen, von denen der erste Infrarotsensor 13a im Bereich des ersten und im Bereich des zweiten Wellenlängenbereichs sensitiv ist und jeweils einen ersten Absorptionswert $A_{CO2}$ im ersten und einen zweiten Absorptionswert $A_{ref}$ im zweiten Wellenlängenbereich liefert. Der zweite Infrarotsensor 13b ist im Bereich des zweiten und des dritten Wellenlängenbereichs sensitiv und liefert einen zweiten Absorptionswert $A_{ref}$ im zweiten Wellenlängenbereich und einen dritten Absorptionswert $A_n$ im dritten Wellenlängenbereich. Der erste Infrarotsensor 13a und die erste Infrarotquelle 12a sind derart angeordnet, dass das Licht der ersten Infrarotquelle 12a auf den ersten Infrarotsensor 13a strahlt. Der zweite Infrarotsensor 13b und die zweite Infrarotquelle 12b sind derart angeordnet, dass das Licht der zweiten Infrarotquelle 12b auf den zweiten Infrarotsensor 13b strahlt. Die Strahlengänge der von den Infrarotsensoren 12a, 12b abgegebenen Lichtstrahlen stehen im vorliegenden Ausführungsbeispiel, wie in **Fig. 4** dargestellt, normal zueinander. Bevorzugt sind die Messbereiche der beiden Filter 17a und 17 b geteilt. Beide ermöglichen die Ermittlung von zwei Absorptionswerten aus An $A_{ref}$ $A_{CO2}$ in den jeweiligen Messbereichen $\lambda_{S,ref} \pm \Delta\lambda_{ref}$, $\lambda_{S,CO2} \pm \Delta\lambda_{CO2}$ und $\lambda_{S,n} \pm \Delta\lambda_n$,

Im vorliegenden Ausführungsbeispiel ermitteln beide Infrarotsensoren 13a, 13b jeweils einen zweiten Absorptionswert $A_{ref}$. Um Schwankungen der Helligkeit der Infrarotquellen 12a, 12b zueinander auszugleichen, wird das Verhältnis zwischen dem vom ersten Infrarotsensor 13a ermittelten zweiten Absorptionswert und dem vom zweiten Infrarotsensor 13b ermittelten zweiten Absorptionswert ermittelt. Multipliziert man den vom zweiten Infrarotsensor 13b ermittelten zweiten Absorptionswert mit dem ermittelten Verhältnis, so erhält man den vom ersten Infrarotsensor 13a ermittelten zweiten Absorptionswert. Damit unterschiedliche Beleuchtungsstärken durch die beiden Infrarotquellen 12a, 12b keinen Einfluss auf das Verhältnis der einzelnen Absorptionswerte zueinander haben, wird der vom zweiten Infrarotsensor 13b ermittelte dritte Absorptionswert mit dem ermittelten Verhältnis, multipliziert und den weiteren Berechnungen zugrunde gelegt.

In **Fig. 5** ist ein typisches Spektrum des Absorptionskoeffizienten gesättigten wässrigen $CO_2$-Lösung dargestellt. Deutlich zu erkennen ist, dass die Absorption im Bereich von etwa 4260 nm deutlich ansteigt, während $CO_2$-freies

Wasser in diesem Wellenlängenbereich eine Erhöhung der Absorption nicht aufweist.

**Fig. 6** zeigt schematisch Absorptionsspektren von vier verschiedenen Flüssigkeiten. Es werden kurz die einzelnen Einflüsse von $CO_2$ und einer den Brechungsindex hebenden und in der jeweiligen Flüssigkeit gelösten Substanz mit einer Konzentration von $C_{Extrakt}$, im vorliegenden Fall von Zucker, auf das Absorptionsspektrum einer ATR-Messung dargestellt. Es sind Absorptionsspektren der folgenden Flüssigkeiten dargestellt.

**Tabelle 1.** $CO_2$-Konzentration und BRIX der Flüssigkeiten, deren Absorptionsspektren in **Fig. 6** dargestellt sind.

|  | $CO_2$[g/l] | $C_{Extrakt}$ [BRIX] |
|---|---|---|
| $S_1$ | 0 | 0 |
| $S_2$ | 5 | 0 |
| $S_3$ | 5 | 10 |
| $S_4$ | 0 | 10 |

Das Absorptionsspektrum $S_1$ einer ersten Flüssigkeit weist einen BRIX-Wert von 0 auf und ist frei von $CO_2$, ist im Wellenlängenbereich zwischen 3000 nm und 4500 nm annähernd konstant und weist referenziert auf den Wasserhintergrund insbesondere keine markanten Steigungen, Maxima, Minima usw. auf.

Ein zweites Absorptionsspektrum $S_2$ wurde für eine zweite Flüssigkeit erstellt, die keinen Zucker enthält und einen BRIX-Wert von 0 und einen Gehalt von 5 g/l $CO_2$ aufweist. Das Absorptionsspektrum $S_2$ der zweiten Flüssigkeit weist bei einer Wellenlänge $\lambda_{CO2}$ von etwa 4260 nm ein deutliches Maximum auf. Unterhalb einer Wellenlänge $\lambda_{ref}$ von etwa 4050 nm entspricht das Absorptionsspektrum $S_2$ der zweiten Flüssigkeit dem Absorptionsspektrum $S_1$ der ersten Flüssigkeit.

Ein drittes Absorptionsspektrum $S_3$ wurde für eine dritte Flüssigkeit erstellt, die Zucker enthält und einen BRIX-Wert von 10 aufweist und zudem einen Gehalt von 5 g/l $CO_2$ aufweist. Im Wellenlängenbereich oberhalb einer Wellenlänge $\lambda_{ref}$ von etwa 4050 nm weist das dritte Absorptionsspektrum $S_3$ einen ähnlichen Verlauf wie das zweite Absorptionsspektrum $S_2$ auf, wobei das Maximum ebenfalls im Bereich einer Wellenlänge $\lambda_{CO2}$ von etwa 4260 nm liegt, jedoch stärker ausgeprägt ist als beim zweiten Absorptionsspektrum $S_2$, d. h. die Absorption stärker ist als beim zweiten Absorptionsspektrum $S_2$. Unterhalb einer Wellenlänge $\lambda_{ref}$ von etwa 4050 nm steigt die Absorption gegenüber den ersten beiden Absorptionsspektren $S_1$, $S_2$ mit fallender Wellenlänge an. Bei einer Wellenlänge $\lambda_n$ von etwa 3800 nm ergibt sich bereits eine deutliche Abweichung zwischen dem Absorptionskoeffizienten der zweiten und der dritten Flüssigkeit aufgrund der Absorption des Extraktes.

Das vierte Absorptionsspektrum $S_4$ wurde für eine vierte Flüssigkeit erstellt, die Zucker enthält und einen BRIX-Wert von 10 aufweist, jedoch im Gegensatz zur dritten Flüssigkeit kein $CO_2$ enthält. Im Bereich oberhalb einer Wellenlänge von $\lambda_{ref}$ von etwa 4050 nm bis zu einem Wellenlängenbereich von etwa 5000 nm weist das Absorptionsspektrum $S_4$ einen näherungsweise parabolischen Verlauf auf. Im Bereich unterhalb einer Wellenlänge von $\lambda_{ref}$ von etwa 4050 nm bis zu einem Wellenlängenbereich von etwa 3000 nm entspricht das Absorptionsspektrum $S_4$ der vierten Flüssigkeit näherungsweise dem Absorptionsspektrum $S_3$ der dritten Flüssigkeit.

Den dargestellten Absorptionsspektren $S_1$, $S_2$, $S_3$, $S_4$ kann entnommen werden, dass sowohl die Änderung des Brechungsindex der jeweiligen Flüssigkeit als auch eine Änderung des $CO_2$-Gehalts der jeweiligen Flüssigkeit Auswirkungen auf das jeweilige Absorptionsspektrum $S_1$, $S_2$, $S_3$, $S_4$ haben und eine Messung alleine bei der Wellenlänge der maximalen Absorption $\lambda_{CO2}$ sowie bei der Wellenlänge von $\lambda_{ref}$ keine eindeutige Bestimmung des $CO_2$-Gehalts zulassen, da der Einfluss des Brechungsindex auf das jeweilige Absorptionsspektrum zu Verfälschungen des Ergebnisses führt. Aus den Absorptionsspektren $S_3$, $S_4$ der dritten und vierten Flüssigkeit kann jedoch entnommen werden, dass eine Korrektur des Einflusses des jeweiligen Brechungsindex durch Bestimmung eines zusätzlichen Absorptionswerts bei einer Wellenlänge $\lambda_n$ im Bereich von 3300 bis 3900 nm korrigiert werden kann.

Bevorzugt wird eine Messung des Absorptionswerts mit einer möglichst scharfen Wellenlängenbegrenzung vorgenommen. Der erste Absorptionswert $A_{CO2}$ wird bei einer vorgegebenen im ersten Wellenlängenbereich liegenden ersten Wellenlänge $\lambda_{CO2}$ (bevorzugt bei 4260 nm) ermittelt. Der zweite Absorptionswert $A_{ref}$ wird bei einer vorgegebenen im zweiten Wellenlängenbereich liegenden zweiten Wellenlänge $\lambda_{ref}$ (bevorzugt bei 4020 nm) ermittelt. Der dritte Absorptionswert $A_n$ wird bei einer vorgegebenen im dritten Wellenlängenbereich liegenden dritten Wellenlänge $\lambda_n$ (bevorzugt bei 3800 nm) ermittelt.

Die in den ATR-Sensoren verwendeten wellenlängenselektiven Mittel besitzen real endliche Halbwertbreiten. Eine spektrale Auflösung wie beispielsweise in Spektrometern ist damit nicht erreichbar. Die am Detektor gemessenen Absorptionswerte entsprechen daher immer der integrierten Intensität über einen Wellenlängenbereich, der durch die Charakteristik des Sensors gekennzeichnet ist. Die Absorption wird also mit einer bestimmten spektralen Breite $\pm \Delta\lambda$ um die Schwerpunktswellenlänge $\lambda$ des Sensors bestimmt.

Um eine vorteilhafte Korrektur durchführen zu können, ist es nicht erforderlich die Absorption bei konkreten fest vorgegebenen Wellenlängen zu ermitteln, vielmehr reicht es aus, wenn die Schwerpunktswellenlänge des jeweiligen Sensors in den jeweiligen Wellenlängenbereichen, d.h. die erste Schwerpunktswellenlänge $\lambda_{CO2}$ liegt im Wellenlängenbereich zwischen 4200 und 4300 nm, die zweite Schwerpunktswellenlänge $\lambda_{ref}$ liegt im Wellenlängenbereich zwischen 3950 nm bis 4050 nm und die dritte Schwerpunktswellenlänge $\lambda_n$ liegt im Wellenlängenbereich zwischen 3300 und 3900 nm, Die jeweiligen Absorptionswerte $\mathbf{A}_{CO2}$, $\mathbf{A}_{ref}$ und $\mathbf{A}_n$ werden durch Messung der integralen absorbierten Intensität im Wellenlängenbereich $\pm \Delta\lambda$ um die jeweiligen Schwerpunktswellenlängen $A_{CO2}$, $\lambda_{reh}$ $\lambda_n$ gemessen. Bevorzugt wird die spektrale Breite $\Delta\lambda$ um die Schwerpunktwellenlänge $\lambda$ geringer als $\pm$ 50 nm sein.

Bevorzugt werden die einzelnen Wellenlängen innerhalb dem vorgegebenen ersten, zweiten und dritten Wellenlängenbereich so gewählt, dass ein ausreichendes Signal-Rausch-Verhältnis für die jeweils zu vermessenden Reststoffe in der Lösung zu erwarten ist.

Im Folgenden werden vier Ausführungsbeispiele beschrieben, wie aus den Messwerten für den ersten, zweiten und dritten Absorptionswert $\mathbf{A}_{CO2}$, $\mathbf{A}_{ref}$, $\mathbf{A}_n$ sowie für die Temperatur $\mathbf{T}$ jeweils ein Wert $\mathbf{c}_{CO2}$ für die $CO_2$-Konzentration ermittelt wird. In der Auswerteeinheit 4, der die einzelnen Absorptionswerte $\mathbf{A}_{CO2}$, $\mathbf{A}_{ref}$, $\mathbf{A}_n$ sowie ein Wert für die Temperatur $\mathbf{T}$ zugeführt sind, wird mittels Anwendung einer Modellfunktion M der Wert $\mathbf{c}_{CO2}$ für die $CO_2$-Konzentration ermittelt.

In allen Ausführungsbeispielen wird eine Formulierung verwendet, bei der die drei Absorptionswerte $\mathbf{A}_{CO2}$, $\mathbf{A}_{ref}$, $\mathbf{A}_n$ jeweils durch die folgenden beiden Differenzwerte $AD_n$, $AD_{CO2}$ ersetzt werden. Der zweite Absorptionswert $\mathbf{A}_{ref}$ wird bei der zweiten Wellenlänge $\lambda_{ref}$ als Bezugswert herangezogen, es werden lediglich die Differenzen zu diesem Bezugswert für die weiteren Berechnungen herangezogen:

$$AD_n = \mathbf{A_n} - \mathbf{A_{ref}}, \qquad AD_{CO2} = \mathbf{A_{CO2}} - \mathbf{A_{ref}}.$$

Generell wird eine Modellfunktion M festgelegt, die die $CO_2$-Konzentration in Abhängigkeit von den beiden Differenzwerten sowie von der Temperatur T festlegt:

$$c_{CO2} = M(AD_{CO2}, AD_n, T).$$

Alternativ zu den beiden Differenzwerten $AD_{CO2}$, $AD_n$ können auch die tatsächlichen Absorptionswerte $\mathbf{A}_{ref}$, $\mathbf{A}_n$, $\mathbf{A}_{CO2}$ verwendet werden. Die Modellfunktion hat dann die folgende Form

$$c_{CO2} = M(A_{CO2}, A_n, A_{ref}, T).$$

Je nach gewünschter Genauigkeitsklasse können mehr oder weniger Terme in der jeweiligen Näherung Berücksichtigung finden, wobei die Modellfunktion jeweils durch eine Anzahl von Kalibrierkonstanten festgelegt wird. Unterschiedliche Modellfunktionen, wie beispielsweise eine Taylorreihenentwicklung können hier zur Modellierung der Zusammenhänge und Abhängigkeiten verwendet werden.

Aufgrund der Betrachtungen zu **Fig. 6** werden die Auswirkungen unterschiedlicher Einflüsse auf den gemessenen Absorptionswert $\mathbf{A}_{CO2}$ sowie auf den ersten Differenzwert $AD_{CO2}$ dargestellt: Der gemessene Absorptionswert $\mathbf{A}_{CO2}$ ist von der Temperatur $\mathbf{T}$, vom $CO_2$-Gehalt $C_{CO2}$ sowie vom Brechungsindex abhängig. In den einzelnen Ausführungsbeispielen werden die folgenden Grundannahmen getroffen, um diese Abhängigkeiten rechnerisch zu berücksichtigen. Eine Modellierung dieses Zusammenhangs kann allgemein folgendermaßen vorgenommen werden:

$AD_{CO2} \sim T$: Für den Zusammenhang zwischen dem gemessenen Absorptionswert $CO_2$ und der Temperatur $\mathbf{T}$ wird ein linearer Ansatz verwendet.

$AD_{CO2} \sim \mathbf{c}_{CO2}$: Für den Zusammenhang zwischen dem ersten Differenzwert $AD_{CO2}$ und dem $CO_2$-Gehalt können unterschiedliche Annahmen getroffen werden, insbesondere kann ein linearer, polynomieller oder ein exponentieller Ansatz verwendet werden.

$AD_{CO2} \sim n$, $AD_n$, $A_n$: Zur Berücksichtigung des Einflusses des Brechungsindex n oder des dritten Absorptionswerts $A_n$ oder des zweiten Differenzwerts $AD_n$ auf den ersten Absorptionswert $AD_{CO2}$ wird bevorzugt ein polynomieller Ansatz gewählt.

Im **ersten Ausführungsbeispiel,** findet die Temperatur $\mathbf{T}$ der Flüssigkeit zusätzlich Berücksichtigung, da die ermittelten Absorptionswerte neben dem Brechungsindex auch von der Temperatur $\mathbf{T}$ der Flüssigkeit abhängen und die Temperatur **T aufgrund** ihrer Auswirkung auf die Dichte der Flüssigkeit und auf die Messanordnung selbst, etwa durch die Änderung der Strahlintensität der Quelle, Auswirkungen auf die ermittelten Absorptionswerte zeigt. Die Auswirkungen der Temperatur $T$ der auf den Differenzwert $AD_{CO2}$ werden in erster Näherung linear berücksichtigt. Es wird angenommen, dass sich der Differenzwert $AD_{CO2}$ als Summe von zwei Termen $f_T(T)$, $f_{CO2}(C_{CO2})$ abhängig ist, die voneinander unabhängig sind.

$$AD_{CO2} = f_T(T) + f_{CO2}(c_{CO2}) = k_0 + k_{T\_1} \cdot T + k_{CO2\_1} \cdot c_{CO2}$$

Hierbei werden einige Modellparameter $k_0$, $k_{T\_1}$, $k_{CO2\_1}$ vorgegeben, die eine Anpassung der Modellfunktion M an die tatsächlichen Messwerte ermöglicht. Durch Umformung ergibt sich für die $CO_2$-Konzentration $c_{CO2}$ unter Messung von Temperatur **T** und dem ersten Differenzwert $AD_{CO2}$

$$c_{CO2,unkorr} = \frac{AD_{CO2} - k_0 - k_{T\_1} \cdot T}{k_{CO2\_1}}$$

Dieses Ergebnis berücksichtigt nicht die Einflüsse des Brechungsindex n auf den ersten Differenzwert $AD_{CO2}$ sowie auf den ersten Absorptionswert $A_{CO2}$. Um eine Berücksichtigung vorzunehmen, kann die nicht korrigierte $CO_2$-Konzentration $c_{CO2,unkorr}$ mit einem Korrekturterm $f_n(AD_n, T)$ multipliziert werden, der vom zweiten Differenzwert $AD_n$ polynomiell und von der Temperatur linear abhängt:

$$f_n(AD_n, T) = A' + B' \cdot T + C' \cdot AD_n + D' \cdot AD_n^2 + E' \cdot AD_n^3$$

Durch Einsetzen des Korrekturterms $f_{korr}(AD_n, T)$ in

$$c_{CO2} = M(AD_{CO2}, AD_n, T) = c_{CO2,unkorr} \cdot f_n(AD_n, T)$$

ergibt sich der folgenden Zusammenhang:

$$M(AD_{CO2}, AD_n\, T) = c_{CO2,korr} =$$
$$= c_{CO2,unkorr} \cdot (A' + B' \cdot T + C' \cdot AD_n + D' \cdot AD_n^2 + E' \cdot AD_n^3 + \cdots)$$

Das Modell weist bei einer polynomiellen Entwicklung des Korrekturterms mit einem Grad von drei insgesamt acht Modellparameter, im vorliegenden Ausführungsbeispiel sind dies A', B', C', D', E' sowie $k_0$, $k_{T\_1}$, $k_{CO2\_1}$.

Zur Bestimmung der Modellparameter wird eine Anzahl von m Messungen der Temperatur **T**, sowie der Absorptionswerte $A_{ref}$, An, $A_{CO2}$ von unterschiedlichen bekannten Flüssigkeiten mit bekannten $CO_2$-Konzentrationen mit jeweils unterschiedlichen Extrakt-Konzentrationen oder Brechungsindizes jeweils bei verschiedenen Temperaturen durchgeführt. Mit einem Fitting-Verfahren werden anhand der ermittelten Messwerte **T**, $A_{ref}$, **A**n, $A_{CO2}$ Modellparameter ermittelt, für die die Modellfunktion bei Anwendung auf die ermittelten Messwerte möglichst gut mit den bekannten $CO_2$-Konzentrationen übereinstimmt.

Alternativ zu einem Fitting-Verfahren können die einzelnen Modellparameter selbstverständlich unter Zugrundelegung der einzelnen Messwerte sowie der bekannten $CO_2$-Konzentrationen $C_{CO2,1}$, $C_{CO2,2}$, ... $C_{CO2,m}$ analytisch gelöst werden.

$$M(T_1, A_{ref,1}, A_{n,1}, A_{CO2,1}) = c_{CO2,1}$$

$$M(T_2, A_{ref,2}, A_{n,2}, A_{CO2,2}) = c_{CO2,2}$$

.....

$$M(T_m, A_{ref,m}, A_{n,m}, A_{CO2,m}) = c_{CO2,m}$$

Ein **zweites Ausführungsbeispiel** der Erfindung berücksichtigt die sich ändernde Eindringtiefe $d_p$ des Messstrahls in die zu prüfende Flüssigkeit, die sich durch den unterschiedlichen Brechungsindex der zu prüfenden Flüssigkeit ergibt. Diese Einflüsse werden nunmehr durch Einführen des zweiten Differenzwerts $AD_n = A_n - A_{ref}$ berücksichtigt. Betrachtet man die für die $CO_2$-Messung angeführten prinzipiellen Zusammenhänge zwischen Konzentration c, Eindringtiefe $d_p$ bzw. untersuchte Schichtdicke des evaneszenten Feldes und dem ersten Differenzwert $AD_{CO2}$ so gilt näherungsweise

$$AD_{CO2} = \varepsilon \cdot c_{CO2} \cdot d_p$$

,

wobei $\varepsilon$ der Durchlässigkeit der jeweiligen Flüssigkeit für Strahlung bei der Messwellenlänge $\lambda_{CO2}$ entspricht. Für die einzelne Messung mit dem Reflexionselement 11 und definierter Strahlgeometrie bei einer bestimmten Wellenlänge $\lambda$ ergibt sich somit ein einfacher Zusammenhang zwischen Brechungsindex und Eindringtiefe $d_p$.

Im dritten Wellenlängenbereich $\lambda_n$ zwischen 3300 nm bis 3900 nm überlagern sich die Absorptionsbereiche von Ethanol (Alkohol) und unterschiedlichen Zuckerarten in wässriger Lösung. Diese Einflüsse werden berücksichtigt, indem der dritte Absorptionswert $A_n$ bei $\lambda_n$ gemessen wird und ein zweiter Differenzwert $AD_n$ zwischen dem dritten und zweiten Absorptionswert ermittelt wird: $AD_n = A_n - A_{ref}$.

Für den zweiten Differenzwert $AD_n$ wird näherungsweise die folgende Annahme getroffen, wobei auch die Temperaturabhängigkeit des Messsignals berücksichtigt wird, sodass sich der folgende Zusammenhang ergibt:

$$AD_n = f_T(T) + f_{ex}(c_{Extrakt}) = \cdot j_0 + j_{T\_1} \cdot T + j_{n\_1} \cdot c_{Extrakt}$$

,

wobei $c_{Extrakt}$ die Konzentration von jeweils in der Flüssigkeit gelösten Extrakten wie beispielsweise Zucker oder Alkoholen angibt, die zu einer Änderung des Brechungsindex der zu prüfenden Flüssigkeit beitragen. Wiederum werden zudem Modellparameter $j_0$, $j_{T\_1}$ und $j_{n\_1}$ vorgegeben, die eine Anpassung der jeweiligen Funktionen f an die tatsächlichen Messwerte ermöglichen. Formt man diese Beziehung nach $c_{Extrakt}$ um, so erhält man den folgenden Zusammenhang.

$$c_{Extrakt} = \frac{AD_n - j_0 - j_{T\_1} \cdot T}{j_{n\_1}}$$

Die damit bestimmbare Extrakt-Konzentration $c_{Extrakt}$ ist ein direktes Maß für den Brechungsindex der Lösung und damit für die Eindringtiefe des Messstrahls in die Lösung. Die Extrakt-Konzentration $c_{Extrakt}$ steht damit als Korrekturfaktor für die $CO_2$-Konzentrationsbestimmung zur Verfügung. Die Extrakt-Konzentration $c_{Extrakt}$ kann allerdings nicht mehr streng linear nach dem Lambert-Beer'schen Gesetz berücksichtigt werden, da dieser Zusammenhang nur näherungsweise für die Absorption durch nur eine einzige bestimmende Komponente im Extrakt gilt. Da aber in der Realität aber eine Mischung bzw. Zusammensetzung aus mehreren Extrakt-Komponenten und/oder Alkohol vorliegt, ist die Mischung damit nicht mehr ein ternäres Stoffgemisch, die physikalischen Verhältnisse lassen sich somit nur näherungsweise darstellen.

Der Brechungsindex ist definiert als Verhältnis der Ausbreitungsgeschwindigkeit des Lichtes im Vakuum c zur Lichtgeschwindigkeit in der Flüssigkeit v und hängt direkt von der Extrakt-Konzentration der zu untersuchenden Flüssigkeit ab, dieses Verhalten wird beispielsweise auch zur Bestimmung der Zuckerkonzentration in Lösungen mittels Refraktometern verwendet. Darüber hinaus steht der Brechungsindex des Stoffgemisches aufgrund der weiteren Abhängigkeiten (Molmasse, Polarisierbarkeit) nicht analytisch aus der bloßen Messung einer Wellenlänge zur Verfügung.

Die an einer Position im ersten Wellenlängenbereich zwischen 3300 nm bis 3900 nm gemessenen Absorptionswerte sind jedoch für die jeweilige Extrakt- und Alkohol-Konzentration repräsentativ.

Die Eindringtiefe $d_p$ wird im Folgenden so modelliert, dass je nach gewünschter Genauigkeit für die weitere Betrachtung die Eindringtiefe $d_p$ bzw. der Brechungsindex n aus der Absorption $AD_n$ mit mehreren Gliedern und Kalibrierkonstanten berücksichtigt wird. Für diese Korrektur kann man nun beispielsweise einen mehrgliedrigen polynomiellen Ansatz wählen oder einen exponentiellen Ansatz, der die gemessene $CO_2$-Konzentration mit der gemessenen Extrakt-Absorption korrigiert.

Verwendet man beispielsweise einen polynomiellen Ansatz für die Eindringtiefe $d_p$, so gilt:

$$d_p = A + B \cdot T + C \cdot AD_n + D \cdot AD_n^2 + E \cdot AD_n^3 + \cdots$$

Damit ergibt sich für die Abhängigkeit der gemessenen Absorption $AD_{CO2}$ einer beliebigen $CO_2$-Konzentration von der Eindringtiefe $d_p$

$$AD_{CO2} = \varepsilon \cdot c_{CO2} \cdot d_p = \varepsilon \cdot c_{CO2} \cdot (A + B \cdot T + C \cdot AD_n + D \cdot AD_n^2 + E \cdot AD_n^3 + \cdots)$$

Formt man diese Gleichung nach $c_{CO2}$ um, so erhält man

$$c_{CO2} = M(AD_{CO2}, AD_n, T) = \frac{AD_{CO2}}{\varepsilon \cdot (A + B \cdot T + C \cdot AD_n + D \cdot AD_n^2 + E \cdot AD_n^3 + \cdots)}$$

Ein **drittes Ausführungsbeispiel** einer Modellfunktion M nimmt auf Messverfahren Rücksicht, bei denen der erste Absorptionswert $AD_{CO2}$ nicht bei einer konkreten Wellenlänge sondern als integral gemessener Absorptionswert über einen bestimmten Wellenlängenbereich von etwa 50nm bis 100 nm ermittelt worden ist.

Wählt man zur Erhöhung der Intensitäten, bei gleichzeitiger Verwendung möglichst günstiger Bauelemente, Filter mit größerer spektraler Breite, gilt der grundsätzlich nach dem Lambert-Beer'schen Gesetz dargestellte lineare Zusammenhang zwischen Konzentration und Intensität der $CO_2$-Absorption nicht mehr. Vielmehr kann dieser Zusammenhang besser durch eine exponentielle Abhängigkeit angenähert werden, wenn die Durchlässigkeit der verwendeten Filter und damit die integral gemessenen Absorption über einen bestimmten Wellenlängenbereich von etwa 50 nm bis 100 nm anstelle der Absorptionsintensität bei einer einzelnen aus dem Spektrum ausgeblendeten Wellenlänge gemessen wird. Bei etwa 40 nm Halbwertsbreite des $CO_2$-Absorptions-Peaks ist also der durchlässige spektrale Wellenlängenbereich des Filters größer oder gleich der tatsächlichen Breite des $CO_2$-Absorptions-Peaks.

$$AD_{CO2} = f(c_{CO2}) = A_{CO2} - A_{Ref} = k_{CO2\_0}^* + k_{CO2\_1}^* \cdot \exp\left(k_{CO2\_2}^* \cdot c_{CO2}\right)$$

bzw. unter Mitberücksichtigung der Temperatur

$$AD_{CO2} = f_T(T) + f(c_{CO2}) = k_0^* + k_{T\_1}^* \cdot T + k_{CO2\_1}^* \cdot \exp\left(k_{CO2\_2}^* \cdot c_{CO2}\right)$$

$$c_{CO2,unkorr} = c_{CO2} = \frac{1}{k_{CO2\_2}^*} \ln\left(\frac{AD_{CO2} - k_0^* - k_{T\_1}^* \cdot T}{k_{CO2\_1}^*}\right)$$

Führt man die Brechungsindexkorrektur analog dem zweiten Ausführungsbeispiel der Erfindung durch, so erhält man

$$M\left(AD_{CO2}, AD_n\, T\right) = c_{CO2,korr} =$$
$$= c_{CO2,unkorr} \cdot \left(A'' + B'' \cdot T + C'' \cdot AD_n + D'' \cdot AD_n^2 + E'' \cdot AD_n^3 + \cdots\right)$$

Ein **viertes Ausführungsbeispiel** einer Modellfunktion M berücksichtigt, dass die einzelnen Gleichungen für Brechungsindex, Temperatur und gemessene Intensitäten bei der $CO_2$-Absorption nicht unabhängig voneinander betrachtet werden können. Der Brechungsindex zeigt ein von der jeweiligen zu untersuchenden Flüssigkeit abhängiges Dispersionsverhalten, ebenso verändert sich der Extinktionskoeffizient in der Gleichung mit der Wellenlänge und Temperatur **T**. Um die komplexe analytische Auswertung der einzelnen Gleichungen nicht durchführen zu müssen, kann vereinfacht der folgende Ansatz gewählt werden:

Ausgehend von einem empirischen Kurvenfit für die $CO_2$-Konzentration werden in einer Modellbildung die Temperatur **T** und der Brechungsindex bzw. damit auch die Eindringtiefe des Messstrahles durch Variation der Konstanten modellgemäß berücksichtigt.

Für den exponentiellen Ansatz bedeutet dies

$$AD_{CO2} = Y_0 + A_1 \cdot \exp\left(-\frac{c_{CO2}}{t_1}\right)$$

Nunmehr wird die Abhängigkeit von der Temperatur **T** und dem Brechungsindex in den einzelnen Termen $Y_0$, $A_1$ und $t_1$ jeweils durch einen linearen Ansatz für die Temperatur **T** und einen polynomiellen Ansatz für den Brechungsindex bzw. für die gemessene Absorption wie folgt berücksichtigt:

$$Y_0 = A_{nY0} + B_{nY0}.AD_n + C_{nY0} \cdot AD_n^2 + A_{TY0} + B_{TY0} \cdot T = A_{Y0} + B_{nY0} \cdot AD_n + C_{nY0} \cdot AD_n^2 + B_{TY0} \cdot T$$

$$A_1 = A_{nA1} + B_{nA1} \cdot AD_n + C_{nA1} \cdot AD_n^2 + A_{TA1} + B_{TA1}.T = A_{A1} + B_{nA1} \cdot AD_n + C_{nA1} \cdot AD_n^2 + B_{TA1}.T$$

$$t_1 = A_{nt1} + B_{nt1} \cdot AD_n + C_{nt1} \cdot AD_n^2 + A_{Tt1} + B_{Tt1} \cdot T = A_{t1} + B_{nt1} \cdot AD_n + C_{nt1} \cdot AD_n^2 + B_{Tt1} \cdot T$$

Formt man die vorstehende Gleichung um, so erhält man als Modellfunktion M den folgenden Ausdruck.

$$M(AD_{CO2}, AD_n, T) = c_{CO2} = -t_1 \cdot \ln\left(\frac{AD_{CO2} - Y_0}{A_1}\right)$$

Durch Verwendung eines linearen Ansatzes für die Temperatur **T** und eines polynomiellen Ansatzes für den Brechungsindex bzw. für die gemessene Absorption ergibt sich für die Modellfunktion M der folgende Zusammenhang

$$c_{CO2} = -\left(A_{t1} + B_{nt1} \cdot AD_n + C_{nt1} \cdot AD_n^2 + B_{Tt1} \cdot T\right)\ln\left(\frac{AD_{CO2} - \left(A_{Y0} + B_{nY0} \cdot AD_n + C_{nY0} \cdot AD_n^2 + B_{TY0} \cdot T\right)}{A_{A1} + B_{nA1} \cdot AD_n + C_{nA1} \cdot AD_n^2 + B_{TA1}.T}\right)$$

, wobei die folgenden Größen $A_{Y0}$, $A_{A1}$, $A_{t1}$, $B_{nY0}$, $B_{nA1}$ $B_{nt1}$, $C_{nY0}$, $C_{nA1}$, $C_{nt1}$, $B_{TY0}$, $B_{TA1}$, $B_{Tt1}$ als Modellparameter auftreten.

Die Bestimmung der Modellparameter erfolgt, wie auch bei den übrigen Ausführungsbeispielen der Erfindung durch Kalibrierung anhand von bekannten Messwerten. Es wird eine Anzahl von m Messungen der Temperatur **T**, sowie der Absorptionswerte $A_{ref}$, **A**n, $A_{CO2}$ von unterschiedlichen bekannten Flüssigkeiten mit bekannten $CO_2$-Konzentrationen mit jeweils unterschiedlichen Extrakt-Konzentrationen oder Brechungsindizes jeweils bei verschiedenen Temperaturen ermittelt. Mit einem Fitting-Verfahren werden anhand der ermittelten Messwerte **T**, $A_{ref}$, **A**n, $A_{CO2}$ Modellparameter ermittelt, für die die Modellfunktion bei Anwendung auf die ermittelten Messwerte möglichst gut mit den bekannten

$CO_2$-Konzentrationen übereinstimmt. Es wird ein Gleichungssystem erstellt, das für jede separate Messung jeweils eine Gleichung aufweist.

$$M(T_1, A_{ref,1}, A_{n,1}, A_{CO2,1}) = c_{CO2,1}$$

$$M(T_2, A_{ref,2}, A_{n,2}, A_{CO2,2}) = c_{CO2,2}$$

$$....$$

$$M(T_m, A_{ref,m}, A_{n,m}, A_{CO2,m}) = c_{CO2,m}$$

Dieses Gleichungssystem wird näherungsweise gelöst. Insbesondere kann durch die Erhöhung der Anzahl der Messungen ein genaueres Ergebnis erzielt werden. Um im vorliegenden Fall sind 12 Modellparameter zu bestimmen, sodass zumindest 12 Gleichungen anzusetzen sind. Im vorliegenden Fall werden 18 Messungen vorgenommen, wobei jeweils die Temperatur T, der Brechungsindex sowie der vorab bekannte $CO_2$-Gehalt in **Tabelle 2** eingetragen sind.

**Tabelle 2** zeigt ein Umsetzungsbeispiel für die Messung der Konstanten mit einem Prototypen des Sensorkopfs. Bei der Messung sind folgende Zustände einzustellen:

| T [°C] | $C_{CO2}$[g/l] | $C_{Extrakt}$ [BRIX] |
|---|---|---|
| 0 ÷ 5 | 1 ÷ 2 | 0 |
| 0 ÷ 5 | 5.5 ÷ 6.5 | 0 |
| 0 ÷ 5 | 10 ÷ 11 | 0 |
| 20 ÷ 25 | 1 ÷ 2 | 0 |
| 20 ÷ 25 | 5.5 ÷ 6.5 | 0 |
| 20 ÷ 25 | 10 ÷ 11 | 0 |
| 0 ÷ 5 | 1 ÷ 2 | 6 ÷ 7 |
| 0 ÷ 5 | 5.5 ÷ 6.5 | 6 ÷ 7 |
| 0 ÷ 5 | 10 ÷ 11 | 6 ÷ 7 |
| 20 ÷ 25 | 1 ÷ 2 | 6 ÷ 7 |
| 20 ÷ 25 | 5.5 ÷ 6.5 | 6 ÷ 7 |
| 20 ÷ 25 | 10 ÷ 11 | 6 ÷ 7 |
| 0 ÷ 5 | 1 ÷ 2 | 14 ÷ 15 |
| 0 ÷ 5 | 5.5 ÷ 6.5 | 14 ÷ 15 |
| 0 ÷ 5 | 10 ÷ 11 | 14 ÷ 15 |
| 20 ÷ 25 | 1 ÷ 2 | 14 ÷ 15 |
| 20 ÷ 25 | 5 ÷ 6 | 14 ÷ 15 |
| 20 ÷ 25 | 9 ÷ 10 | 14 ÷ 15 |

Bei der Kalibrierung werden also zumindest 3 verschiedene $CO_2$-Konzentrationen bei jeweils mindestens 2 verschiedenen Temperaturen und jeweils mindestens 3 verschiedenen Zuckerkonzentrationen (und damit Brechungsindizes n) gemessen. Daraus ergeben sich die 12 Modellparameter für den gewählten analytischen Ansatz.

Diese Auswertung der vorgenommenen Kalibriermessungen für die bekannten $CO_2$-Konzentrationen ergibt folgende Konstanten:

$$A_{y0} = -0.01983, B_{ny0} = -6.4902, C_{ny0} = -28.04101, B_{Ty0} = -0.00245$$

$$A_{A1} = 0.09595, B_{nA1} = 4.72755, C_{nA1} = 20.50155, B_{TA1} = 0.00224$$

$$A_{t1} = 14.83044, B_{nt1} = 26.58616, C_{nt1} = 679.05946, B_{Tt1} = -0.30869$$

Die tatsächliche Berechnung der Konzentration erfolgt dann aus den Differenzwerte $AD_n$ und $AD_{CO2}$ und der Temperatur T, verwendet man die angegebenen Modellparameter, stimmen die mit der erfindungsgemäßen Ausführung gemessenen $CO_2$-Werte mit Vergleichsmessungen ausgezeichnet überein.

Bei sämtlichen Modellfunktionen kann die Abhängigkeit von der Temperatur vernachlässigt werden, indem eine vorab vorgegebene Temperatur festgelegt wird, die die jeweilige Flüssigkeit typischerweise bei der Messung der Absorptionswerte aufweist. Die Bestimmung der Modell-Parameter kann in einem solchen Fall auch dann erfolgen, wenn Flüssigkeiten von einer einzigen Temperatur, die vorzugsweise der vorgegebenen Temperatur entspricht, verwendet werden. Diese Flüssigkeiten brauchen sich somit nur im $CO_2$-Gehalt sowie im Extraktgehalt $C_{Extrakt}$ unterscheiden.

**Patentansprüche**

1. Verfahren zur Bestimmung des $CO_2$-Gehalts ($C_{CO2}$) in einer zu prüfenden Flüssigkeit, insbesondere in einem Getränk,

    - wobei die Messung der Absorption der zu messenden Flüssigkeit bei zumindest einer Wellenlänge ($\lambda_{CO2}$) innerhalb eines ersten Wellenlängenbereichs zwischen 4200 und 4300 nm durchgeführt wird und ein erster Absorptionswert ($\mathbf{A}_{CO2}$) mittels der Methode der abgeschwächten Totalreflexion (ATR) gemessen wird,
    - wobei die Messung der Absorption der zu messenden Flüssigkeit bei zumindest einer zweiten Wellenlänge ($\lambda_{ref}$) innerhalb eines zweiten Wellenlängenbereichs zwischen 3950 und 4050 nm durchgeführt wird und ein zweiter Absorptionswert ($\mathbf{A}_{ref}$) mittels der Methode der abgeschwächten Totalreflexion (ATR) gemessen wird,

    **dadurch gekennzeichnet,**

    - **dass** die Messung der Absorption der zu messenden Flüssigkeit zusätzlich bei zumindest einer dritten Wellenlänge ($\lambda_n$) innerhalb eines dritten Wellenlängenbereichs zwischen 3300 und 3900 nm durchgeführt wird, und ein dritter Absorptionswert ($\mathbf{A}_n$) mittels der Methode der abgeschwächten Totalreflexion (ATR) gemessen wird,
    - **dass** eine vorgegebene Modellfunktion (M) zur Ermittlung des $CO_2$-Gehalts ($C_{CO2}$) auf Grundlage des ersten, zweiten und dritten Absorptionswerts herangezogen wird, und
    - **dass** die Modellfunktion (M) auf den ermittelten ersten, zweiten und dritten Absorptionswerts ($\mathbf{A}_{CO2}$, $\mathbf{A}_{ref}$, $\mathbf{A}_n$) angewendet wird und das Ergebnis der Auswertung als $CO_2$-Gehalt ($c_{CO2}$) der zu prüfenden Flüssigkeit zur Verfügung gehalten wird.

2. Verfahren nach Anspruch 1, **dadurch gekennzeichnet,**

    - **dass** die Messung des ersten Absorptionswerts ($\mathbf{A}_{CO2}$) durch Bestimmung der absorbierten Intensität in einem ersten Messbereich vorgenommen wird, der durch eine im ersten Wellenlängenbereich liegende erste Schwerpunktswellenlänge ($\lambda_{S,CO2}$) sowie eine erste Bereichsbreite $2\Delta\lambda_{CO2}$ festgelegt ist, wobei der erste Messbereich im Bereich von $A_{S,CO2} \pm \Delta\lambda_{CO2}$ liegt, und/oder
    - **dass** die Messung des zweiten Absorptionswerts ($\mathbf{A}_{ref}$) durch Bestimmung der absorbierten Intensität in einem zweiten Messbereich vorgenommen wird, der durch eine im zweiten Wellenlängenbereich liegende zweite Schwerpunktswellenlänge ($\lambda_{S,ref}$) sowie eine zweite Bereichsbreite $2\Delta\lambda_{ref}$ festgelegt ist, wobei der zweite Messbereich im Bereich von $\lambda_{S,ref} \pm \Delta\lambda_{ref}$ liegt, und/oder
    - **dass** die Messung des dritten Absorptionswerts ($\mathbf{A}_n$) durch Bestimmung der absorbierten Intensität in einem dritten Messbereich vorgenommen wird, der durch eine im dritten Wellenlängenbereich liegende dritte Schwerpunktswellenlänge ($\lambda_{S,n}$) sowie eine dritte Bereichsbreite $2\Delta\lambda_n$ festgelegt ist, wobei der dritte Messbereich im Bereich von $\lambda_{s,n} \pm \Delta\lambda_n$ liegt, wobei die erste und/oder zweite und/oder dritte Bereichsbreite ($2\Delta\lambda_{CO2}$, $2\Delta\lambda_{ref}$, $2\Delta\lambda_n$) jeweils in einem Bereich zwischen 20 nm und 200 nm, insbesondere bei 100 nm liegt.

3. Verfahren nach einem Anspruch 1, **dadurch gekennzeichnet,**

    - **dass** der erste Absorptionswert ($\mathbf{A}_{CO2}$), vorzugsweise ausschließlich, bei einer vorgegebenen im ersten Wel-

lenlängenbereich liegenden ersten Wellenlänge ($\lambda_{CO2}$), von vorzugsweise 4260 nm, ermittelt wird, und/oder
- **dass** der zweite Absorptionswert ($A_{ref}$), vorzugsweise ausschließlich, bei einer vorgegebenen im zweiten Wellenlängenbereich liegenden zweiten Wellenlänge ($\lambda_{ref}$), von vorzugsweise 4020 nm, ermittelt wird, und/oder
- **dass** der dritte Absorptionswert ($A_n$), vorzugsweise ausschließlich, bei einer vorgegebenen im dritten Wellenlängenbereich liegenden dritten Wellenlänge ($A_n$), von vorzugsweise 3800 nm, ermittelt wird.

4. Verfahren nach einem der vorangehenden Ansprüche, **dadurch gekennzeichnet, dass** zusätzlich zur Bestimmung des ersten, zweiten und dritten Absorptionswerts ($A_{CO2}$, $A_{ref}$, $A_n$) auch die Temperatur (T) der zu prüfenden Flüssigkeit ermittelt wird,

- dass die Modellfunktion (M) zur Ermittlung des $CO_2$-Gehalts ($c_{CO2}$) neben dem ersten, zweiten und dritten Absorptionswert ($A_{CO2}$, $A_{ref}$, $A_n$) auch die Temperatur (**T**) der zu prüfenden Flüssigkeit berücksichtigt, und
- dass die Modellfunktion (M) auf den ermittelten ersten, zweiten und dritten Absorptionswerts ($A_{CO2}$, $A_{ref}$, $A_n$) sowie auf die ermittelte Temperatur (**T**) angewendet wird und das Ergebnis der Auswertung als $CO_2$-Gehalt ($c_{CO2}$) der zu prüfenden Flüssigkeit zur Verfügung gehalten wird.

5. Verfahren nach einem der vorangehenden Ansprüche, **dadurch gekennzeichnet, dass** vor der Bestimmung des $CO_2$-Gehalts ($c_{CO2}$) eine Modellfunktion (M) erstellt und für die Bestimmung des $CO_2$-Gehalts ($c_{CO2}$) zur Verfügung gehalten wird, indem

- eine Vielzahl von Referenzmessungen des ersten, zweiten und dritten Absorptionswerts für ($A_{CO2}$, $A_{ref}$, $A_n$) jeweils unterschiedliche Referenzflüssigkeiten mit bekanntem $CO_2$-Gehalt ($c_{CO2}$) mit unterschiedlichem, gegebenenfalls bekannten Brechungsindex, durchgeführt werden,
- mittels eines Fitting-Verfahrens die Modellfunktion (M) der Form

$$M = M(\mathbf{A_{CO2}}, \mathbf{A_{ref}}, \mathbf{A_n}, ..., B_1, ..., B_N)$$

erstellt wird, wobei vorab unbekannte Modellparameter ($B_1$, ..., $B_N$) an den jeweils vorgegebenen $CO_2$-Gehalt ($c_{CO2}$) sowie die ermittelten ersten, zweiten und dritten Absorptionswerte ($A_{CO2}$, $A_{ref}$, An) angepasst werden, sodass man bei Anwendung der Modellfunktion (M) auf die ersten, zweiten und dritten Absorptionswerte ($A_{CO2}$, $A_{ref}$, $A_n$) jeweils, zumindest näherungsweise, den bekannten $CO_2$-Gehalt ($c_{CO2}$) erhält.

6. Verfahren nach Anspruch 5, **dadurch gekennzeichnet, dass** bei der Vielzahl von Referenzmessungen jeweils neben dem ersten, zweiten und dritten Absorptionswert ($A_{CO2}$, $A_{ref}$, $A_n$) auch die Temperatur (T) der jeweiligen Referenzflüssigkeit ermittelt wird,

- mittels eines Fitting-Verfahrens die Modellfunktion (M) der Form

$$M = M(\mathbf{A_{CO2}}, \mathbf{A_{ref}}, \mathbf{A_n}, T, C_1, ..., C_N)$$

erstellt wird, wobei vorab unbekannte Modellparameter ($C_1$, ..., $C_N$) an den jeweils vorgegebenen $CO_2$-Gehalt ($c_{CO2}$), die ermittelten ersten, zweiten und dritten Absorptionswerte ($A_{CO2}$, $A_{ref}$, $A_n$) sowie die jeweilige Temperatur (**T**) angepasst werden, sodass man bei Anwendung der Modellfunktion (M) auf die ersten, zweiten und dritten Absorptionswerte ($A_{CO2}$, $A_{ref}$, $A_n$) sowie die Temperatur (**T**) jeweils, zumindest näherungsweise, den bekannten $CO_2$-Gehalt ($c_{CO2}$) erhält.

7. Vorrichtung zur Bestimmung des $CO_2$-Gehalts ($c_{CO2}$) in einer zu prüfenden Flüssigkeit, umfassend

- eine erste ATR-Messeinheit (1) zur Bestimmung eines ersten Absorptionswerts ($A_{CO2}$) bei zumindest einer ersten Wellenlänge ($A_{CO2}$) innerhalb eines ersten Wellenlängenbereichs zwischen 4200 und 4300 nm,
- eine zweite ATR-Messeinheit (2) zur Bestimmung eines zweiten Absorptionswerts ($A_{ref}$) bei zumindest einer zweiten Wellenlänge ($\lambda_{ref}$) innerhalb eines zweiten Wellenlängenbereichs zwischen 3950 und 4050 nm,

**gekennzeichnet durch**

- eine dritte ATR-Messeinheit (3) zur Bestimmung eines dritten Absorptionswerts ($A_n$) bei einer zumindest dritten Wellenlänge innerhalb eines dritten Wellenlängenbereichs ($\lambda_n$) zwischen 3300 und 3900 nm, und

- eine Auswerteeinheit (4), die den ATR-Messeinheiten (1, 2, 3) nachgeschaltet ist und der die Resultate der ATR-Messeinheiten (1, 2, 3) zugeführt sind, wobei die Auswerteeinheit (4) eine Modellfunktion (M) auf den ersten, zweiten und dritten Absorptionswert ($A_{CO2}$, $A_{ref}$, $A_n$) anwendet und das Ergebnis der Auswertung an ihrem Ausgang als $CO_2$-Gehalt ($c_{CO2}$) der zu prüfenden Flüssigkeit zur Verfügung hält.

8. Vorrichtung nach Anspruch 7, **dadurch gekennzeichnet, dass** die ATR-Messeinheiten (1, 2, 3) für die Bestimmung der absorbierten Intensität in einem ersten, zweiten und dritten Messbereich sensitiv sind,

- wobei der erste Messbereich durch eine im ersten Wellenlängenbereich liegende erste Schwerpunktswellenlänge ($\lambda_{S,CO2}$) sowie eine erste Bereichsbreite $2\Delta\lambda_{CO2}$ festgelegt ist, und der erste Messbereich im Bereich von $\lambda_{S,CO2} \pm \Delta\lambda_{CO2}$ festgelegt ist, und/oder

- wobei der zweite Messbereich durch eine im zweiten Wellenlängenbereich liegende zweite Schwerpunktswellenlänge ($\lambda_{S,ref}$) sowie eine zweite Bereichsbreite $2\Delta\lambda_{ref}$ festgelegt ist, und der zweite Messbereich im Bereich von $\lambda_{S,ref} \pm \Delta\lambda_{ref}$ festgelegt ist, und/oder

- wobei der dritte Messbereich durch eine im dritten Wellenlängenbereich liegende dritte Schwerpunktswellenlänge ($\lambda_{S,n}$) sowie eine dritte Bereichsbreite $2\Delta\lambda_n$ festgelegt ist, und der dritte Messbereich im Bereich von $\lambda_{s,n} \pm \Delta\lambda_n$ festgelegt ist,

wobei die erste und/oder zweite und/oder dritte Bereichsbreite ($2\Delta\lambda_{CO2}$, $2\Delta\lambda_{ref}$, $2\Delta\lambda_n$) jeweils in einem Bereich zwischen 20 nm und 200 nm, insbesondere bei 100 nm liegt.

9. Vorrichtung nach einem der Ansprüche 7 oder 8, **gekennzeichnet durch** einen der Auswerteeinheit (4) vorgeschalteten Temperaturfühler (5) zur Bestimmung der Temperatur (T) der zu prüfenden Flüssigkeit, wobei die Auswerteeinheit (4) eine Modellfunktion (M) auf den ersten, zweiten und dritten Absorptionswert ($A_{CO2}$, $A_{ref}$, $A_n$) sowie auf die vom Temperaturfühler (5) ermittelte Temperatur (**T**) anwendet und das Ergebnis der Auswertung an ihrem Ausgang als $CO_2$-Gehalt ($c_{CO2}$) der zu prüfenden Flüssigkeit zur Verfügung hält.

10. Vorrichtung nach einem der Ansprüche 7 bis 9, **gekennzeichnet durch** einen Behälter (6) zur Aufbewahrung oder Durchleitung der zu prüfenden Flüssigkeit, wobei die sensitiven Oberflächenteile der ATR-Messeinheiten (1, 2, 3) und gegebenenfalls auch des Temperaturfühlers (5) bei Befüllen oder Durchströmen des Behälters (6) mit der zu prüfenden Flüssigkeit mit dieser in Kontakt treten und insbesondere im Inneren des Gefäßes (6) angeordnet sind.

11. Vorrichtung nach einem der Ansprüche 7 bis 10, **dadurch gekennzeichnet,**

- **dass** in der Auswerteeinheit (4) Speicher für vorgegebene Koeffizienten ($B_1$, $B_2$, ...) vorgesehen sind, und

- **dass** die Auswerteeinheit (4) eine Recheneinheit aufweist, der die abgespeicherten Koeffizienten ($B_1$, $B_2$, ...) sowie der erste, zweite und dritte Absorptionswert ($A_{CO2}$, $A_{ref}$, $A_n$), sowie gegebenenfalls auch die ermittelte Temperatur (**T**), zugeführt sind und die auf Grundlage der ihr zugeführten Werte die Modellfunktion (M) auswertet und am Ausgang der Auswerteeinheit (4) zur Verfügung hält.

12. Vorrichtung nach einem der Ansprüche 7 bis 11, **dadurch gekennzeichnet,**

- **dass** jede ATR-Messeinheit (1, 2, 3) jeweils ein ATR-Reflexionselement, eine ATR-Infrarotquelle, und einen ATR-Infrarotsensor umfassen, oder

- **dass** die ATR-Messeinheiten (1, 2, 3) ein gemeinsames ATR-Reflexionselement und eine gemeinsame im ersten, zweiten und dritten Wellenlängenbereich aktive ATR-Infrarotquelle aufweisen und einen gemeinsamen, im ersten, zweiten und dritten Wellenlängenbereich aktiven ATR-Infrarotsensor aufweisen, wobei im Strahlengang zwischen der ATR-Infrarotquelle und dem ATR-Infrarotsensor ein einstellbarer Filter, insbesondere ein Filterrad oder ein Fabry-Perot-Interferometer, vorgesehen ist, der je nach seiner Einstellung jeweils nur für Strahlung im ersten, zweiten oder dritten Wellenlängenbereich durchlässig ist, oder

- **dass** die ATR-Messeinheiten (1, 2, 3) ein gemeinsames ATR-Reflexionselement und eine gemeinsame im ersten, zweiten und dritten Wellenlängenbereich aktive ATR-Infrarotquelle aufweisen und für den ersten, zweiten und dritten Wellenlängenbereich jeweils separate, am Ende des jeweiligen Strahlengangs befindliche ATR-Infrarotsensoren vorgesehen sind, oder

- **dass** die ATR-Messeinheiten (1, 2, 3) ein gemeinsames ATR-Reflexionselement und einen gemeinsamen für alle Wellenlängenbereiche sensitiven ATR-Infrarotsensor aufweisen, und für den ersten, zweiten und dritten Wellenlängenbereich jeweils separate, ATR-Infrarotquellen vorgesehen sind.

**13.** Vorrichtung nach einem der Ansprüche 7 bis 12, **dadurch gekennzeichnet, dass** die ATR-Messeinheiten (1, 2a, 2b, 3) zumindest zwei separate ATR-Infrarotquellen (12a, 12b) und zugehörige ATR-Infrarotsensoren (13a, 13b) aufweisen, die jeweils voneinander unabhängige Strahlengänge besitzen und für jeweils zwei Messbereiche verschieden sensitiv sind, wobei jeweils eine Messeinheit (2a) des ersten Infrarotsensors (13a) und jeweils eine Messeinheit (2b) des zweiten Infrarotsensors (13b) jeweils für denselben Wellenlängenbereich sensitiv sind und eine Referenzierungseinheit vorgesehen ist, die den Messwert der dritten Messeinheit (3) mit dem Verhältnis der Messwerte der beiden für denselben Wellenlängenbereich sensitiven Messeinheiten (2a, 2b) multipliziert und an ihrem Ausgang zur Verfügung hält.

Fig. 1

Fig.2

Fig 3

13b, 17b

11

13a, 17a

14

12a

12b

Fig. 4

EP 2 629 093 A1

FIG. 5

22

Fig. 6

**Europäisches Patentamt**
**European Patent Office**
**Office européen des brevets**

## EUROPÄISCHER RECHERCHENBERICHT

Nummer der Anmeldung

EP 13 45 5002

### EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | DE 10 2005 048807 B3 (JOHANN WOLFGANG GOETHE UNI [DE]) 16. November 2006 (2006-11-16) * Zusammenfassung * * Absätze [0034], [0037], [0039], [0049] * * Ansprüche 17,18 * ----- | 7-13 | INV. G01N33/14 G01N21/55 G01N21/03 G01N21/35 G01N21/31 |
| X | DE 28 09 910 A1 (DIESSEL GMBH & CO) 22. März 1979 (1979-03-22) | 7-13 | |
| Y | * Ansprüche 1-4 * ----- | 1-6 | |
| A | "PUTTING THE RIGHT FIZZ IN BEER", CHEMICAL ENGINEER, INSTITUTION OF CHEMICAL ENGINEERS, LONDON, GB, Bd. 498, 13. Juni 1991 (1991-06-13), Seite 18, XP002041431, ISSN: 0302-0797 * Spalte 1, Zeile 1 - Spalte 2, Zeile 25 * ----- | 1-13 | |
| Y | EP 1 965 193 A1 (ANTON PAAR GMBH [AT]) 3. September 2008 (2008-09-03) * Absatz [0014] - Absatz [0066]; Abbildung 1 * ----- | 1-6 | RECHERCHIERTE SACHGEBIETE (IPC) G01N |

Der vorliegende Recherchenbericht wurde für alle Patentansprüche erstellt

| Recherchenort | Abschlußdatum der Recherche | Prüfer |
|---|---|---|
| München | 10. Mai 2013 | Consalvo, Daniela |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument

& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 13 45 5002

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

10-05-2013

| Im Recherchenbericht angeführtes Patentdokument | Datum der Veröffentlichung | Mitglied(er) der Patentfamilie | | Datum der Veröffentlichung |
|---|---|---|---|---|
| DE 102005048807 B3 | 16-11-2006 | DE | 102005048807 B3 | 16-11-2006 |
| | | WO | 2007041999 A1 | 19-04-2007 |
| DE 2809910 A1 | 22-03-1979 | DE | 2809910 A1 | 22-03-1979 |
| | | DK | 64579 A | 09-09-1979 |
| | | GB | 2018423 A | 17-10-1979 |
| | | US | 4228192 A | 14-10-1980 |
| EP 1965193 A1 | 03-09-2008 | AT | 504436 A4 | 15-05-2008 |
| | | EP | 1965193 A1 | 03-09-2008 |
| | | US | 2008218733 A1 | 11-09-2008 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- EP 1630543 A
- AT 409673